# EUROPEAN PATENT SPECIFICATION

(11) **EP 1 379 504 B1**
(45) Date of publication and mention of the grant of the patent: **28.10.2009**
(21) Application number: 02730122.5
(22) Date of filing: 08.04.2002
(51) Int. Cl.: C07D 211/58, C07D 401/14, C07D 211/96, C07D 417/14, A61K 31/4468

(54) **BIPIPERIDINYL-DERIVATIVES AND THEIR USE AS CHEMOKINE RECEPTORS INHIBITORS**
BIPIPERIDIN-DERIVATE UND IHRE VERWENDUNG ALS INHIBITOREN DER CHEMOKINREZEPTOREN
DERIVES BIPIPERIDINYLE ET LEUR UTILISATION COMME INHIBITEURS DES RECEPTEURS DE CHEMOKINE

(30) Priority: 09.04.2001 GB 0108876
(43) Date of publication of application: 14.01.2004
(73) Proprietor: Novartis Pharma GmbH, 1230 Wien (AT)
(72) Inventor: ALBERT, Rainer, CH-4052 Basel (CH); BRUNS, Christian, 79110 Freiburg (DE); NUNINGER, François, F-68270 Wittenheim (FR); STREIFF, Markus, CH-4127 Birsfelden (CH); THOMA, Gebhard, D-79540 Lörrach (DE); ZERWES, Hans-Günter, D-79539 Lörrach (DE)
(74) Representative: Patent- und Rechtsanwaltssozietät Maucher, Börjes & Kollegen
(86) International application number: PCT/EP2002/003871
(87) International publication number: WO 2002/081449

(56) References cited:
- WO-A-00/66559
- WO-A-00/76972
- WO-A-01/98268
- WO-A-02/22592
- WO-A-98/11082
- WO-A-98/11128
- DE-A- 19 643 331

## Description

The present invention relates to piperidine derivatives, process for their production, their uses and pharmaceutical compositions containing them.

More particularly, the present invention provides a compound of formula I 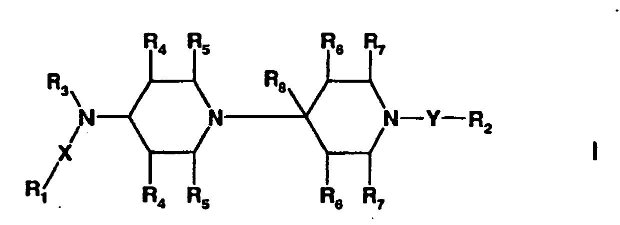 wherein
X is a direct bond; -CH₂-; -CH₂-CH₂-; -CHR₉-; -C(O)-; -O-; -NH- or NR₉;
R₁ is optionally R₁₀ and/or R₁₁-substituted phenyl; optionally R₁₀ and/or R₁₁-substituted heteroaryl; optionally R₁₀ and/or R₁₁-substituted heteroaryl N-oxide; or optionally R₁₀ and/or R₁₁-substituted naphthyl;
R₂ has one of the significances given for R₁; or is optionally R₁₀ and/or R₁₁-substituted fluorenyl; optionally R₁₀-substituted C₁-C₆ alkyl; optionally R₁₀-substituted C₂-C₆ alkenyl; optionally R₁₀-substituted C₃-C₆ cycloalkyl; optionally R₁₀-substituted adamantyl; or optionally R₁₀-substituted C₄-C₈ cycloalkenyl;
R₃ has one of the significances given for R₁; or is optionally R₁₀ and/or R₁₁-substituted fluorenyl;
or 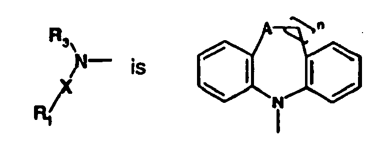 wherein A is -CH₂-, -NH-, -NR₉-, -S-, -SO-, SO₂- or -O-, n is 0, 1 or 2, and the aromatic rings are each, independently optionally R₁₀-substituted;
each of R4, independently, has one of the significances of R₅; or is CN; OH; OR₉; F; Cl; Br; or I;
each of R₅, independently, is H; C₁-C₆ alkyl; C₁-C₆ hydroxyalkyl: C₂-C₆ alkoxyalkyl; C₁-C₆ halogenoalkyl; phenyl; benzyl; or heteroaryl;
each of R₆, independently, has one of the significances given for R₄;
each of R₇, independently, has one of the significances given for R₅;
R₈ is H; C₁-C₆ alkyl; C₂-C₆ alkenyl; C₂-C₆ alkynyl; phenyl; benzyl; CN; CH₂NH₂; CH₂NHR₉; CH₂NR₉R₉; CH₂NHC(O)R₉; CH₂NR₉C(O)R₉; CH₂NHC(O)NHR₉; CH₂NR₉C(O)NHR₉; CH₂NR₉C(O)NR₉R₉; CH₂NHC(O)OR_{9;} CH₂NR₉C(O)OR₉; CH₂NHSO₂R₉; CH₂N(SO₂R₉)₂; or CH₂NR₉SO₂R₉;
each R₉, independently, is C₁-C₆ alkyl; C₃-C₆ cycloalkyl; C₂-C₆ alkenyl; C₂-C₆ alkynyl; phenyl; benzyl; heteroaryl: or CF₃; R₁₀ represents 1 to 4 substituents independently selected from C₁-C₆ alkyl; C₁-C₆ hydroxyalkyl; C₂-C₆ alkoxyalkyl; C₁-C₆ halogenoalkyl; C₃-C₆ cycloalkyl; C₂-C₆ alkenyl; C-C₆ cycloalkenyl; C₂-C₆ alkynyl; phenyl; heteroaryl; heteroaryl N-oxide ; F; Cl; Br; I; OH; OR₉; CONH₂; CONHR₉; CONR₉R₉ ;OC(O)R₉; OC(O)OR₉; OC(O)NHR₉; OC(O)NR₉R₉; OSO₂R₉; COOH; COOR₉; CF₃; CHF₂; CH₂F; CN; NO₂; NH₂; NHR₉; NR₉R₉; NHC(O)R₉; NR₉C(O)R₉; NHC(O)NHR₉; NHC(O)NH₂; NR₉C(O)NHR₉; NR₉C(O)NR₉R₉; NHC(O)OR₉; NR₉C(O)OR₉; NHSO₂R₉; N(SO₂R₉)₂; NR₉SO₂R₉; SR₉; S(O)R₉; SO₂R₉; Si(CH₃)₃ and B(OC(CH₃)₂)₂;
R₁₁ represents two adjacent substituents which form an annulated 4-7 membered nonaromatic ring optionally containing up to two heteroatoms selected independently from N, O and S; and
Y is -C(O)-,
in free form or in salt form.

Any alkyl, alkenyl or alkynyl may be linear or branched. Halogeno is F, Cl, Br or l.

By heteroaryl is meant an aromatic ring system comprising mono-, bi- or tricyclic systems which contains up to 4 heteroatoms independently selected from N, O and S, such as for example furyl, thienyl, pyrrolyl, imidazolyl, pyrazolyl, thiazolyl, isothiazolyl, oxazolyl, isoxazolyl, oxadiazolyl, thiadiazolyl, triazolyl, tetrazolyl, pyridyl, pyridazinyl, pyrimidinyl, pyrazinyl, triazinyl, tetrazinyl, indolyl, benzothiophenyl, benzofuranyl, benzimidazolyl, indazolyl, benzotriazolyl, benzothiazolyl, benzoxazolyl, quinolinyl, isoquinolinyl, phthalazinyl, quinoxalinyl, quinazolinyl, cinnolinyl or naphthyridinyl.

Preferred annulated 4-7membered non-aromatic ring as represented by R₁₁ is annulated 5 or 6 membered non aromatic ring optionally containing 1 or 2 oxygen and include e.g. -O-CH₂-O- or -O-CH₂-CH₂-O-, attached to 2 adjacent carbon atoms.

The compounds of formula I may exist in free form or in salt form, e.g. addition salts with e.g. organic or inorganic acids, for example, hydrochloric acid, acetic acid when R₁, R₂, and /or R₃ comprises an optionally substituted amino group or a heterocyclic residue which can form addition salts. When the compounds of formula I have one or more asymmetric centers in the molecule, e.g. when a piperidine ring is substituted, the present invention is to be understood as embracing the various optical isomers, as well as racemates, diastereoisomers and mixtures thereof.

In the compounds of formula I, the following significances are preferred individually or in any sub-combination:
1. R₁ is optionally R₁₀-substituted phenyl; optionally R₁₀-substituted heteroaryl; or optionally R₁₁-substituted phenyl,
2. R₂ is optionally R₁₀-substituted phenyl; optionally R₁₀-substituted heteroaryl; optionally R₁₀-substituted heteroaryl N-oxide; or optionally R₁₀-substituted naphthyl.
3. R₃ is optionally R₁₀-substituted phenyl; optionally R₁₀-substituted heteroaryl; or optionally R₁₀-substituted naphthyl.
4. Each of R₄, R₅, R₆ or R₇, independently, is H; C₁-C₆ alkyl; or benzyl.
5. R₈ is H; C₁-C₆ alkyl; or C₂-C₆ alkenyl.
6. R₉ is C₁-C₆ alkyl; C₃-C₆ cycloalkyl; C₂-C₆ alkenyl; C₂-C₆ alkynyl; phenyl; benzyl; heteroaryl; or CF₃.
7. R₁₀ represents 1 to 3 substituents independently selected from C₁-C₆ alkyl; C₁-C₆ hydroxyalkyl; C₂-C₆ alkoxyalkyl; C₁-C₈ halogenoalkyl; C₃-C₆ cycloalkyl; C₂-C₆ alkenyl; C₃-C₆ cycloalkenyl; C₂-C₆ alkynyl; phenyl; heteroaryl; heteroaryl N-oxide; F; Cl; Br, I; OH; OR₉; CONH₂; CONHR₉; CONR₉R₉; OC(O)R₉; OC(O)OR₉; OC(O)NHR₉; OC(O)NR₉R₉; OSO₂R₉; COOH; COOR₉; CF₃; CHF₂; CH₂F; CN; NO₂; NH₂; NHR₉; NR₉R₉; NHC(O)R₉; NR₉C(O)R₉; NHC(O)NHR₉; NHC(O)NH₂; _{N}R9C(O)NHR₉; NR₉C(O)NR₉R₉; NHC(O)OR₉; NR₉C(O)OR₉; NHSO₂R₉; N(SO₂R₉)₂; NR₉SO₂R₉; SR₉; S(O)R₉; SO₂R₉ and Si(CH₃)₃.
8. R₁₁ represents -O-CH₂-O- attached on 2 adjacent carbon atoms.
9. X is a direct bond or -CH₂-.

In the preferred compounds of formula I, R₁₀ may represent 1-3 substituents selected from from C₁₋₆alkyl; phenyl; heteroaryl; heteroaryl N-oxide; F; Cl; Br; I; OH; OR₉; CONH₂; CONHR₉; CONR₉R₉; COOH; COOR₉; CF₃; CHF₂; CH₂F; NH₂; NHR₉; NR₉R₉; NHC(O)R₉; NR₉C(O)R₉; NHC(O)NHR₉; NHC(O)NH₂; NR₉C(O)NHR₉; NR₉C(O)NR₉R₉; NHC(O)OR₉ and NR₉C(O)OR₉.

R₉ is preferably C₁-C₆ alkyl; C₃-C₆ cycloalkyl; phenyl; benzyl; or heteroaryl; more preferably C₁-C₆ alkyl.

The present invention also includes a process for the preparation of a compound of formula I which process comprises
a) for the preparation of a compound of formula I wherein X is a direct bond, -CH₂-, -CH₂-CH₂- or -CHR₉- and Y is -CO-, -C(O)CH₂-, -S(O)- or -S(O₂)-, amidating a compound of formula II 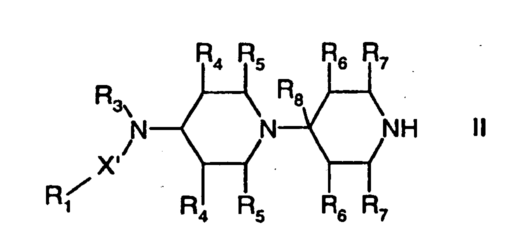 wherein R₁ and R₃ to R₈ are as indicated above and X' is a direct bond, -CH₂-, -CH₂-CH₂- or -CHR₉-
   with a compound of formula III

   R₂-Y'-A' III

   wherein R₂ is as defined above, Y' is -CO-, and A' is a leaving group, e.g. Cl Br or OH,
b) for the preparation of a compound of formula I wherein X is a direct bond and Y is -CH₂-, submitting a compound of formula II as defined above wherein X' is a direct bond, to a reductive amination; or
c) for the preparation of a compound of formula I wherein X is CH₂-, -CH₂-CH₂- or -CHR₉- and Y is -CO-,
   reacting a compound of formula IV 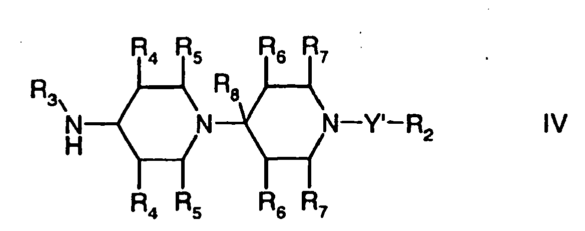 wherein R₂ to R₈ and Y' are as defined above, with a compound of formula V

   R₁-X"-Hal V

   wherein R₁ is as defined above and X" is CH₂- or -CHR₉-;
and, where required, converting the resulting compound of formula I obtained in free form into the desired salt form, or vice versa.

The reaction steps a), b) or c) may be performed in accordance with methods known in the art or as disclosed in the Examples below. When R₈ comprises a group which should not participate in the reaction, this group may be protected in accordance with methods known in the art.

Compounds of formula II, used as starting material may be prepared as follows: 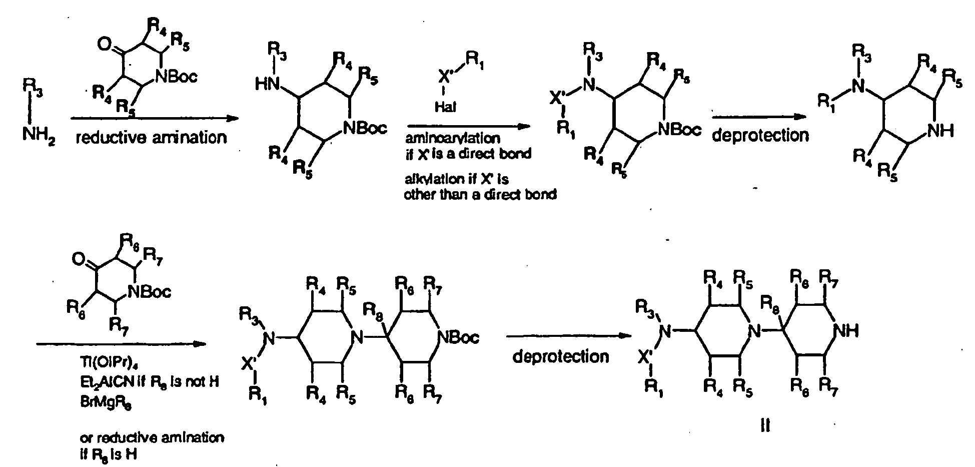 wherein X' and R₁ to R₈ are as defined above and Hal is Cl, Br or I. In above formulae, Boc is a protecting group which means tert.-butyloxycarbonyl. This protecting group may be replaced in above reaction scheme by any amino protecting group, e.g. as disclosed in "Protective Groups in Organic Synthesis" by T. W. Greene, J.Wiley & Sons NY, 2nd ed., Chapter 7, 1991 and references therein, e.g. benzyloxycarbonyl or 9-fluorenylmethoxy carbonyl.

Compounds of formula IV, used as starting material, may be prepared as follows: 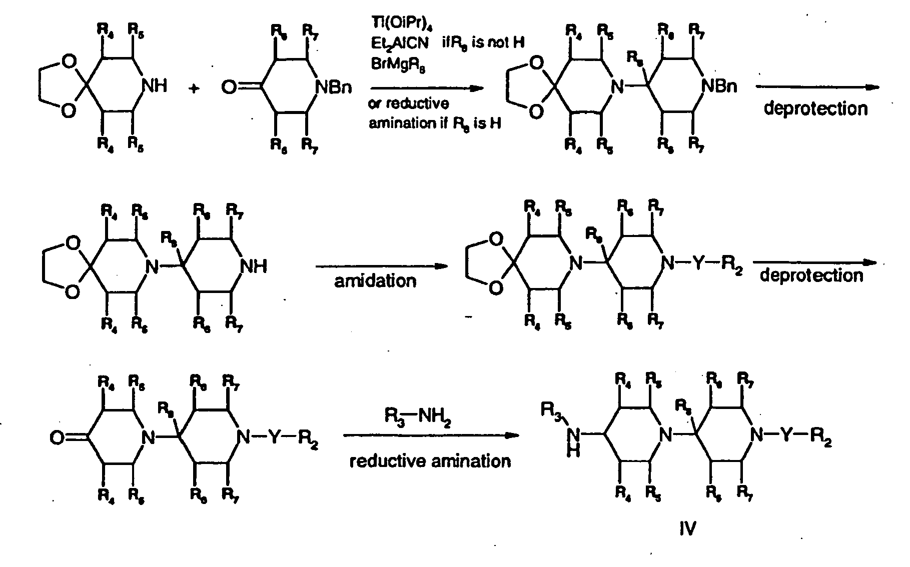 wherein R₂ to R₈ and Y are as defined above and Bn is benzyl.

Above reactions may be carried out in accordance with methods known in the art or as disclosed hereafter.

Insofar as the production of the starting materials is not particularly described, the compounds are known or may be prepared analogously to methods known in the art or as described hereafter.

The following Examples are illustrative of the invention, without limitation. Following abbreviations ara used:
- Bn =: Benzyl
- Boc =: tert.-Butyloxycarbonyl
- DMF =: Dimethylformamide
- DMSO =: Dimethylsufoxide
- BINAP =: 2,2'-Bis(diphenylphosphino)-1,1'-binaphthyl
- THF =: Tetrahydrofuran
- TFA =: Trifluoroacetic acid
- RT =: Room temperature

### Example 1: (2,6-Dimethyl-phenyl)-4-diphenylamino-4'-methyl-[1,4']bipiperidinyl-1'-yl)-methanone

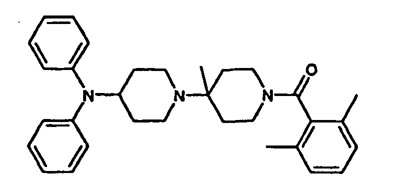

A mixture of (4'-Methyl-[1,4']bipiperidinyl-4-yl)-diphenyl-amine (0.25 g, 0.71 mmol), 2,6-dimethylbenzoic acid (0.32 g, 2.13 mmol), 2-(1H-benzotriazole-1-yl)-1,1,3,3-tetramethyluronium tetrafluoroborate (0.57 g, 1.5 mmol), EtN(i-Pr)₂ (0.6 ml) and DMF (5 ml) is stirred for 16 h at 20°C. The mixture is diluted with t-butyl methylether (25 ml), washed with 2N NaOH (25 ml) and brine (25 ml) and dried with sodium sulfate. The solvent is removed and the residue purified by chromatography (SiO₂, t-butyl methylether/cyclohexane 1:4→1:0). The title compound is isolated as a colorless solid. MS/ESI 482 (M+H)⁺;
¹H NMR (400 MHz, DMSO) δ= 0.89 (3 H, s), 1.14-1.25 (3 H, m), 1.39 (1 H, m), 1.59 (1 H, m), 1.75 (1 H, m), 1.83-1.95 (2 H, m), 2.01 (3 H, s), 2.13 (3 H, s), 2.11-2.24 (2 H, m), 2.85 (2 H, m), 2.95 (1 H, m), 3.01 (1 H, m), 3.35 (1 H, m), 3.70-3.83 (2 H, m), 6.77 (4 H, m), 6.92-7.05 (4 H, m), 7.12 (1 H, m), 7.26 (4 H, m).

(4'-Methyl-[1,4']bipiperidinyl-4-yl)-diphenyl-amine, used as starting material may be prepared as follows:
a) A mixture of phenyl-piperidin-4-yl-amine (4.14 g; 15.0 mmol), iodobenzene (3.06 g; 15.0 mmol), Pd(OAc)₂ (0.14 g; 0.63 mmol); BINAP (0.43 g; 0.69 mmol), t-BuOK (17.5 ml of 1 M solution in THF) in toluene (20 ml) is heated at 110°C for 5 h. The mixture is diluted with ethyl acetate, extracted with sodium hydrogencarbonate and brine and dried with sodium sulfate. The solvent is removed and the residue subjected to chromatography (SiO₂, *t*-butyl methylether /cyclohexane 1:9→1:1). 4-Diphenylamino-piperidine-1-carboxylic acid tert-butyl ester is isolated as a yellow solid. MS/ESI 353 (M+H)⁺
b) A mixture of TFA (5 ml), methylene chloride (5 ml) water (0.25 ml) and 4-diphenylamino-piperidine-1-carboxylic acid tert-butyl ester (1.5 g; 4.2 mmol) is stirred for 2 h at 20°C. Sodium hydroxide (4N) is added and the mixture extracted with ethyl acetate. The organic phase is dried with sodium sulfate and the solvent removed. Diphenyl-piperidin-4-yl-amine is isolated as a colorless oil. MS/ESI 253 (M+H)⁺
c) A suspension of diphenyl-piperidin-4-yl-amine (1.26 g, 5.00 mmol), 1-(tert-butyl oxycarbonyl)-4-piperidone (1.00 g, 5.00 mmol), and titanium(IV) isopropoxide (1.42 g, 5.00 mmol) in 1,2-dichloroethane (25 ml) is stirred for 1 h at 80°C and then for 16 h at 20°C.
   Diethylaluminum cyanide (10 ml 1M solution in toluene) is added and the mixture stirred for additional 24 h. The solvent is removed and the crude material dissolved in tetrahydrofuran (25 ml). Methylmagnesium bromide (8.7 ml 3M solution in ether) is added dropwise and the mixture stirred for 3 h at 20°C. Ammonium chloride (10 % solution, 50 ml) and ethyl acetate (50 ml) are added, the organic phase washed with ammonium chloride (10 % solution, 50 ml) and sodium hydrogencarbonate (10 % solution, 50 ml), dried with sodium sulfate and the solvent removed. The residue is subjected to chromatography (SiO₂, ethyl acetate/cyclohexane 1:9→1:1). 4-Diphenylamino-4'-methyl-[1,4']bipiperidinyl-1'-carboxylic acid tert.-butyl ester is isolated as a colorless solid MS/ESI 450 (M+H)⁺.
d) A mixture of trifluoroacetic acid (2 ml) and water (0.1 ml) is added dropwise to a solution of compound a) above (0.81 g, 1.80 mmol) in methylene chloride (5 ml) and the mixture stirred for 3 h at 20°C. Sodium hydrogencarbonate (10% solution, 10 ml) and ethyl acetate (20ml) are added and the organic phase dried with sodium sulfate. The solvent is removed and the residue sublected to chromatography (RP-18, methanol/H₂O 1:3→0:1). The title compound is isolated as a colorless oil. MS/ESI 350 (M+H)⁺; ¹H NMR (400 MHz, CDCI₃) □= 0.88 (3 H, s), 1.35 (4 H, m), 1.60 (4 H, m), 1.93 (2 H, m), 2.15 (2 H, m), 2.58 (2 H, m), 2.87 (2 H, m), 2.96 (2 H, m), 3.76 (1 H, m), 6.78 (4 H, m), 6.94 (2 H, m), 7.22 (4 H, m).

By following the procedure of Example 1 and using as starting material (4'-methyl-[1,4']bipiperidinyl-4-yl)-diphenyl-amine, the compounds of formula X₁ 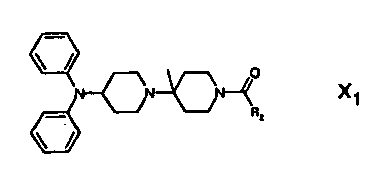 wherein R₂ has the significances as given in Table 1, may be prepared.

**Table 1**

| Example | R₂ | MS/ESI (M+H)⁺ |
|---|---|---|
| 2 | 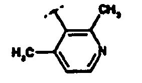 | 483 |
| 3 | 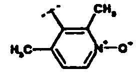 | 499 |
| 4 | 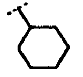 | 460 |
| 5 | 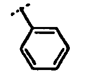 | 454 |
| 6 | 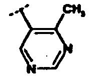 | 470 |
| 7 | 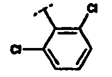 | 522 |
| 8 | 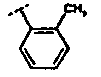 | 468 |
| 9 | 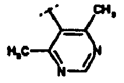 | 484 |
| 10 | 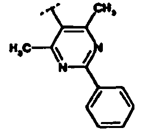 | 560 |
| 11 | 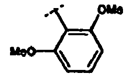 | 514 |
| 12 | 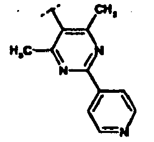 | 561 |
| 13 | 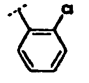 | 488 |
| 14 | 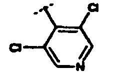 | 523 |
| 15 | 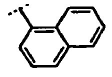 | 504- |
| 16 | 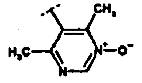 | 500 |
| 17 | 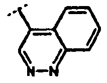 | 506 |
| 18 | 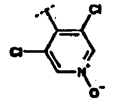 | 539 |
| 19 | 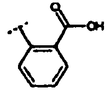 | 498 |
| 20 | 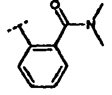 | 525 |
| 21 | 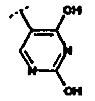 | 488 |
| 22 | 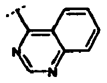 | 506 |
| 23 | 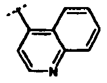 | 505 |
| 24 | 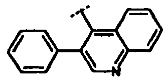 | 581 |
| 25 | 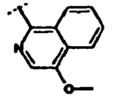 | 535 |
| 26 | 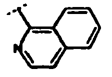 | 505 |
| 27 | 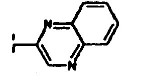 | 506 |
| 28 | 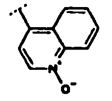 | 521 |
| 29 | 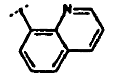 | 505 |
| 30 | 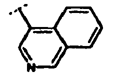 | 505 |
| 31 | 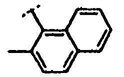 | 518 |
| 32 | 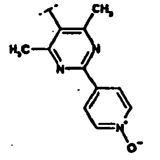 | 577 |
| 33 | 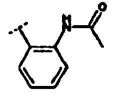 | 511 |
| 34 | 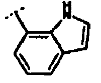 | 493 |
| 35 | 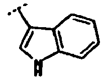 | 493 |
| 36 | 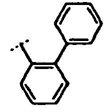 | 530 |
| 37 | 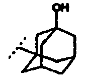 | 528 |
| 38 | 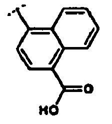 | 548 |
| 39 | 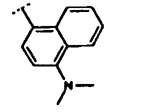 | 547 |
| 40 | 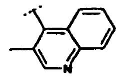 | 519 |

### Reference Example 41: [4'-Methyl-1'-(2,4,6-trimethyl-benzenesulfonyl)-[1,4']bipiperidinyl-4-yl]-diphenyl-amine

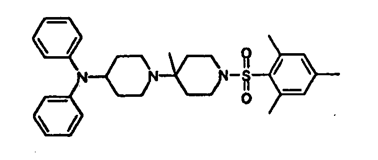

A mixture of (4'-methyl-[1,4']bipiperidinyl-4-yl)-diphenyl-amine (70 mg, 0.20 mmol) and 2,4,6-trimethyl-benzenesulfonyl chloride (65 mg, 0.30 mmol) and diisopropyl ethylamine (0.50 ml) in methylene chloride (3 ml) is stirred for 4h at RT. The mixture is diluted with ethyl acetate, extracted with sodium hydrogencarbonate (10 % solution) and dried with sodium sulfate. The solvent is removed and the residue subjected to chromatography (SiO₂, t-butyl methylether/cyclohexane 1:9→1:0). The title compound is isolated as a colorless solid.
MS/ESI 532 (M+H)⁺

### Reference Example 42: [1'-(2,6-Dimethyl-benzyl)-4'-methyl.[1,4']bipiperidinyi-4-yl]-diphenylamine

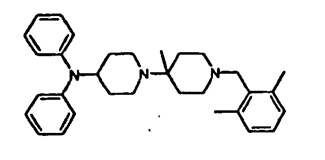

A mixture of (4'-Methyl-[1,4']bipiperidinyl-4-yl)-diphenyl-amine (70 mg, 0.20 mmol) and 2.6-dimethyl-benzaldehyde (34 mg, 0.25 mmol) and Na(OAc)₃BH (53 mg, 0.25 mmol) in 1,2-dichloroethane (10 ml) is stirred at RT for 16 h. The mixture is diluted with ethyl acetate, extracted with sodium hydrogencarbonate (10 % solution) and dried with sodium sulfate. The solvent is removed and the residue subjected to chromatography (SiO₂, tert-butyl methylether/methanol 1:0→10:1). The title compound is isolated as a colorless solid.
MS/ESI 468 (M+H)⁺

### Example 43: (2,6-Dimethyl-phenyl)-4-diphenylamino-[1,4']bipiperidinyl-1'-yl)-methanone

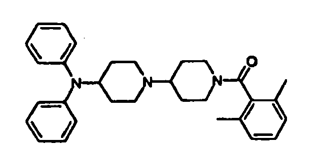

A mixture of TFA salt of [1,4']bipiperidinyl-4-yl-diphenyl-amine (77 mg, 0.23 mmol), 2,6-dimethylbenzoic acid (100 mg, 0.67 mmol), 2-(1H-benzotriazole-1-yl)-1,1,3,3-tetramethyluronium tetrafluoroborate (254 mg, 0.67 mmol), EtN(i-Pr)₂ (2 ml) and DMF (3 ml) is stirred for 5 h at RT. The mixture is diluted with tert-butyl methylether (10 ml), washed with 2N NaOH and brine and dried with sodium sulfate. The solvent is removed and the residue purified by chromatography (SiO₂, *t*-butyl methylether/cyclohexane 1:1→ethyl acetate→ethyl acetate/H₂O 98:2). The title compound is isolated as a colorless solid.
MS/ESI 468 (M+H)⁺
[1,4'] Bipiperidinyl-4-yl-dipherylamine, used as starting materials, may be prepared as follows:
a) A mixture of diphenyl-piperidin-4-yl-amine (1.06 g; 4.2 mmol), 4-oxo-piperidine-1-carboxylic acid tert-butyl ester (1.0 g; 5.0 mmol), AcOH (0.62 g; 10.3 mmol) and Na(OA_{C})₃BH (1.0 g; 4.7 mmol) in 1,2-dichloroethane (15 ml) is stirred for 4h at 65°C. The mixture is diluted with *t-*butyl methylether, extracted with 1 N NaOH and dried with sodium sulfate. The solvent is removed and the residue subjected to chromatography (SiO₂, *t*-butyl methylether/cyclohexane 1:9→1:0). 4-Diphenylamino-[1,4']bipiperidinyl-1'-carboxylic acid tert-butyl ester is isolated as a colorless solid. MS/ESI 436 (M+H)⁺
b) A mixture of 4-Diphenylamino-[1,4']bipiperidinyl-1'-carboxylic acid tert-butyl ester (1.06 g; 2.4 mmol), TFA (2.5 ml), H₂O (0.25 ml) and methylene chloride (5 ml) is stirred at RT for 4 h. The mixture is added dropwise to ether and the precipitate formed is filtered off. The TFA salt of [1,4']bipiperidinyl-4-yl-diphenyl-amine is isolated as a colorless solid. MS/ESI 336 (M+H)⁺

By following the procedure of Example 2 above and using as starting materials [1,4']bipiperidinyl-4-yl-diphenyl-amine the compounds of formula X₂ 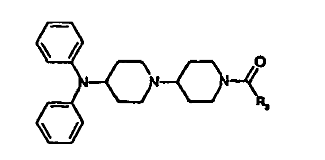 wherein R₂ has one of the significances given in Table 2, may be prepared

**Table 2**

| Example | R₂ | MS/ESI |
|---|---|---|
| 44 | 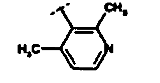 | 469 |
| 45 | 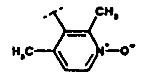 | 485 |
| 46 | 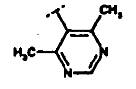 | 470 |
| 47 | 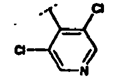 | 509 |
| 48 | 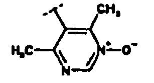 | 486 |
| 49 | 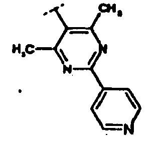 | 547 |
| 50 | 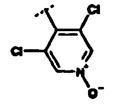 | 525 |

### Example 51: {4-[(4-Bromo-phenyl)-phenyl-amino]-4'-methyl-[1,4']biplperldinyl-1'-yl}-(2,6-dimethyl-phenyl)-methanone

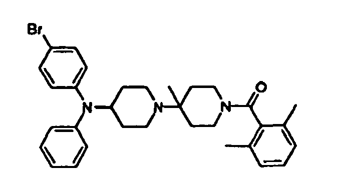

A mixture of [4-(4-bromo-phenylamino)-4'-methyl-[1,4']bipiperidinyl-1'-yl]-(2,6-dimethylphenyl)-methanone (97 mg; 0.20 mmol), iodobenzene (41 mg; 0.20 mmol), Pd (OAc)₂ (1.9 mg; 0.008 mmol), BINAP (5.7 mg; 0.009 mmol) and *t*-BuOK (0.23 ml of 1 M solution on THF) in toluene (3 ml) is heated at 110°C for 16h. The mixture is diluted with ethyl acetate and filtered. The resulting solution is extracted with 2N NaOH and brine and dried with sodium sulfate. The solvent is removed and the residue subjected to chromatography (first SiO₂, t-butyl methylether/cyclohexane 1:4→1:0 and subsequently RP-18, methanol/H₂O 7:3). The title compound is isolated as a colorless solid. MS/ESI 560 (M+H)⁺.

[4-(4-bromo-pherylamino)-4'-methyl-[1,4] bipiperidinyl-1'yl]-2,6-dimethylphenyl)-methanone, used as starting material, may be prepared as follows:
a) 8-(1-Benzyl-4-methyl-piperidin-4-yl)-1,4-dioxa-8-aza-spiro[4.5]decane is prepared from 1,4-dioxa-8-aza-spiro[4.5]decane and 1-benzyl-piperidin-4-one following a procedure as described in example 1c). MS/ESI 331 (M+H)⁺.
b) A mixture of 8-(1-benzyl-4-methyl-piperidin-4-yl)-1,4-dioxa-8-aza-spiro[4.5]decane (2.0 g, 6.1 mmol) and Pd(OH)₂ (20%) on charcoal (1 g) in methanol (30 ml) is hydrogenated for 16h at RT. The catalyst is filtered off and the solvent removed. Crude 8-(4-methyl-piperidin-4-yl)-1,4-dioxa-8-aza-spiro[4.5]decane is isolated as a yellow oil. MS/ESI 241 (M+H)⁺.
c) (2,6-Dimethyl-phenyl)-[4-(1,4-dioxa-8-aza-spiro[4.5]dec-8-yl)-4-methyl-piperidin-1-yl]-methanone is obtained from crude 8-(4-methyl-piperidin-4-yl)-1,4-dioxa-8-aza-spiro[4.5]decane and 2,6-dimethyl-benzoic acid by following a procedure as described in example 1. MS/ESI 373 (M+H)⁺
d) A solution of (2,6-dimethyl-phenyl)-[4-(1,4-dioxa-8-aza-spiro[4.5]dec-8-yl)-4-methyl-piperidin-1-yl]-methanone (915 mg; 2.46 mmol) in dioxan (30 ml) and HCl (6N; 30ml) is stirred for 4h at 50°C. The mixture is diluted with ethyl acetate (50 ml), extracted with 2N NaOH and brine and dried with sodium sulfate. Removal of the solvent affords 1'-(2,6-dimethyl-benzoyl)-4'-methyl-[1,4']bipiperidinyl-4-one is isolated as a colorless solid. MS/ESI 329 (M+H)⁺.
e) A mixture of 1'-(2,6-dimethyl-benzoyl)-4'-methyl-[1,4']bipiperidinyl-4-one (49.3 mg; 0.15 mmol), 4-bromo-phenylamine (29 mg, 0.165 mmol), acetic acid (18 mg; 0.30 mmol) and NaBH(OAC)₃ (35 mg; 0.165 mmol) in (CH₂Cl)₂ (4 ml) is stirred for 16h at RT. The mixture is diluted with ethyl acetate, extracted with 2N NaOH and brine and dried with sodium sulfate. The solvent is removed and the residue subjected to chromatography (RP-18, methanol/H₂O 8:2→1:0). [4-(4-bromo-pherylamino)-4'-methyl-[1,4] bipiperidinyl-1'yl]-2,6-dimethylphenyl)-methanone is isolated as a colorless solid. MS/ESI 484 (M+H)⁺.

### Example 52: {4-[Benzyl-(4-bromo-phenyl)-amino]-4'-methyl-[1,4']bipiperidinyl-1'-yl}-(2,6-dimethyl-phenyl)-methanone

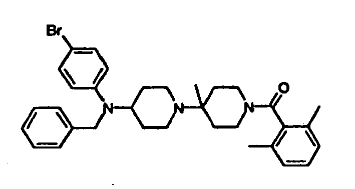

A mixture of {4-[(4-bromo-phenyl)-phenyl-amino]-4'-methyl-[1,4']bipiperidinyl-1'-yl}-(2,6-dimethyl-phenyl)-methanone (97 mg; 0.20 mmol), bromomethyl-benzene (376 mg, 2.2 mmol) and K₂CO₃ (138 mg; 1.0 mmol) in DMF (3 ml) is stirred at 100°C for 16h. The mixture is diluted with ethyl acetate, extracted with 2N NaOH and brine and dried with sodium sulfate. The solvent is removed and the residue subjected to chromatography (first SiO₂, t-butyl methylether and subsequently RP-18, methanol/H₂O 8:2). The title compound is isolated as a colorless solid. MS/ESI 574 (M+H)⁺.

### Example 53: [4-(Benzyl-phenyl-amino)-4'-methyl-[1,4']bipiperidinyl-1'-yl]-(2,6-dimethyl-phenyl)methanone

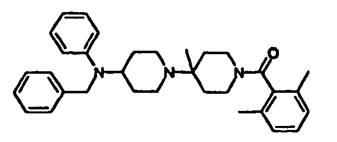

It is prepared from (2,6-dimethyl-phenyl)-(4'-methyl-4-phenylamino-[1,4']bipiperidinyl-1'-yl)-methanone and benzyl bromide following a similar procedure as described in example 52. MS/ESI 496 (M+H)⁺. The starting material may be prepared from 1'-(2,6-dimethyl-benzoyl)-4'-methyl-[1,4']bipiperidinyl-4-one, by following a similar procedure as described in example 51e). MS/ESI 406 (M+H)⁺.

By following the procedure of Example 53 above and using the appropriate starting materials the compounds of formula X₃ 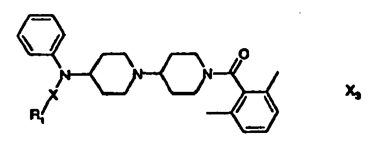 wherein -X-R₁ has the significances as indicated in Table 3 below, may be prepared.

**Table 3**

| Example | -X-R₁ | MS/ESI (M+H)⁺ |
|---|---|---|
| 54 | 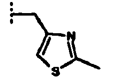 | 517 |
| 55 | 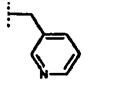 | 497 |

### Example 56: (2,4-Dimethyl-pyridin-3-yl)-{4'-methyl-4-[phenyl-(4-trifluoromethyl-phenyl)-amino]-[1,4']bipiperidinyl-1'-yl}-methanone

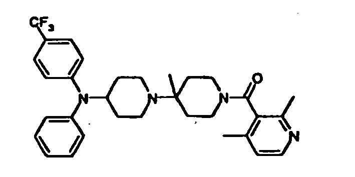

It is prepared from 4-(4-trifluoromethyl-phenylamino)-piperidine-1-carboxylic acid tert-butyl ester by using a procedure as described in example 1. MS/ESI 551 (M+H)⁺. The starting material is prepared from 4-trifluoromethyl-phenylamine and 4-oxo-piperidine-1-carboxylic acid tert-butyl ester following a procedure as described in example 51e). MS/ESI 345 (M+H)⁺.

### Example 57: [4-(Biphenyl-4-yl-phenyl-aminoy)-4'-methyl-[1,4']bipiperidinyl-1'-yl]-(2,6-dimethyl-pheny)-methanone

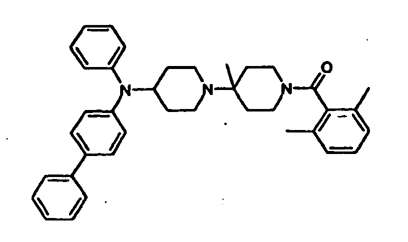

It is prepared from 4-phenylamino-piperidine-1-carboxylic acid tert-butyl ester and 4-bromobiphenyl by using a procedure as described in example 1. MS/ESI 558 (M+H)⁺.

### Example 58: {4-[(4-Bromo-phenyl)-phenyl-amino]-[1,4']bipiperidinyl-1'-yl}-(4,6-dimethyl-pyrimidin-5-yl)-methanone

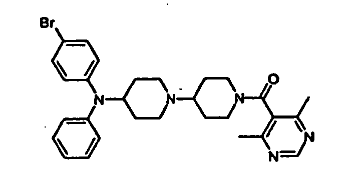

It is prepared from [1,4']bipiperidinyl-4-yl-(4-bromo-phenyl)-phenyl-amine and 4,6-dimethyl-pyrimidine-5-carboxylic acid by following a procedure as described in example 1. MS/ESI 548 (M+H)⁺.

[1,4']bipiperidinyl-4-yl-(4-bromo-phenyl)-phenyl-amine used as starting materials may be prepared as follows:
a) 4-(4-Bromo-phenylamino)-piperidine-1-carboxylic acid tert-butyl ester is prepared from 4-bromo-phenylamine and 4-oxo-piperidine-1-carboxylic acid tert-butyl ester as described in example 51 e). MS/ESI 355 (M+H)⁺.
b) 4-[(4-Bromo-phenyl)-phenyl-amino]-piperidine-1-carboxylic acid tert-butyl ester is prepared from 4-(4-bromo-phenylamino)-piperidine-1-carboxylic acid tert-butyl ester and iodo-benzene as described in example 51. MS/ESI 431 (M+H)⁺.
c) (4-Bromo-phenyl)-phenyl-piperidin-4-yl-amine is prepared from 4-[(4-bromo-phenyl)-phenyl-amino]-piperidine-1-carboxylic acid tert-butyl ester as described in example 1b). MS/ESI 331 (M+H)⁺.
d) 4-[(4-Bromo-phenyl)-phenyl-amino]-[1,4']bipiperidinyl-1'-carboxylic acid tert-butyl ester is prepared from (4-bromo-phenyl)-phenyl-piperidin-4-yl-amine and 4-oxo-piperidine-1-carboxylic acid tert-butyl ester as described in example 43a). MS/ESI 514 (M+H)⁺.
e) [1,4']Bipiperidinyl-4-yl-(4-bromo-phenyl)-phenyl-amine is prepared from 4-[(4-bromophenyl)-phenyl-amino]-[1,4']bipiperidinyl-1'-carboxylic acid tert-butyl ester as described in example 1b). MS/ESI 414 (M+H)⁺.

By using a procedure as disclosed above and the corresponding starting materials, the compounds of formula X₄ 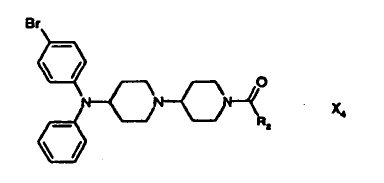 wherein R₂ is as defined in Table 4 below, may be prepared.

**Table 4**

| Example | R₂ | MS/ESI (M+H)⁺ |
|---|---|---|
| 59 | 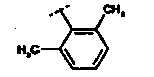 | 546 |
| 60 | 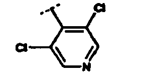 | 587 |
| 61 | 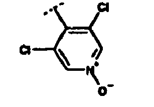 | 603 |
| 62 | 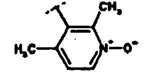 | 563 |
| 63 | 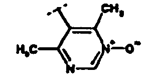 | 564 |
| 64 | 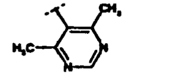 | 548 |
| 65 | 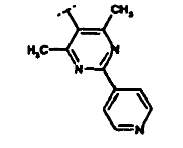 | 625 |
| 66 | 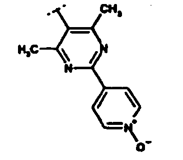 | 641 |

### Example 67: (2,6-Dimethyl-phenyl)-[4-(phenyl-pyridin-3-yl-amino)-[1,4']bipiperidinyl-1'-yl]-methanone

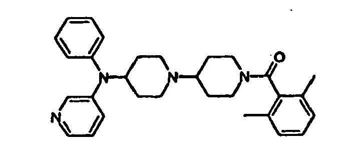

It is prepared from 4-phenylamino-piperidine-1-carboxylic acid tert-butyl ester and 3-bromopyridine by using a procedure as described in example 58 and 58b) to e). MS/ESI 469 (M+H)⁺.

By following a procedure as disclosed above, the compounds of formula X₅ 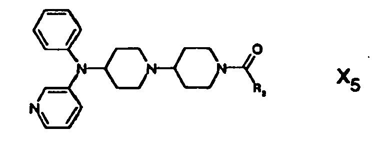 wherein R₂ is as given in Table 5 below, may be prepared.

**Table 5**

| Example | R₂ | MS/ESI (M+H)⁺ |
|---|---|---|
| 68 | 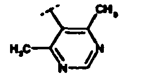 | 471 |
| 69 | 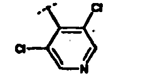 | 510 |
| 70 | 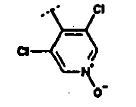 | 526 |
| 71 | 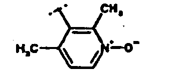 | 486 |
| 72 | 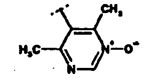 | 487 |

### Example 73: (4,6-Dimethyl-pyrimidin-5-yl)-[4'-methyl-4-(phenyl-pyridin-3-yl-amino)-[1,4']bipiperidinyl-1'-yl]-methanone

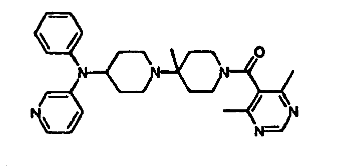

It is prepared from phenyl-piperidin-4-yl-pyridin-3-yl-amine and 4-phenylamino-piperidine-1-carboxylic acid tert-butyl ester using a procedure as described in example 1, 1c) and 1d). MS/ESI 485 (M+H)⁺.

By following the procedure as disclosed in example 73, the compounds of formula X₆ 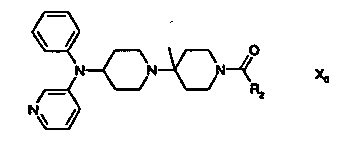 wherein R₂ has the significances as indicated in Table 6, may be prepared.

**Table 6**

| Example | R₂ | MS/ESI (M+H)⁺ |
|---|---|---|
| 74 | 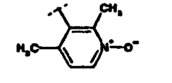 | 500 |
| 75 | 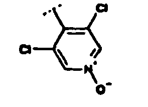 | 540 |
| 76 | 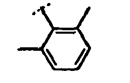 | 483 |

### Example 77: (4-[(4-Bromo-phenyl)-phenyl-amino]-4'-methyl-[1,4']bipiperidinyl-1'-yl}-(4,6-dimethyl-pyrimidin-5-yl)-methanone

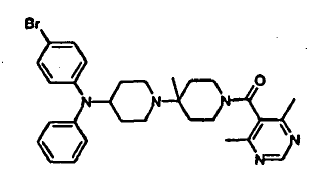

It is prepared from 4-bromo-phenyl)-phenyl-piperidin-4-yl-amine and 4-phenylamino-piperidine-1-carboxylic acid tert-butyl ester using a procedure as described in example 1, 1c) and 1d). MS/ESI 562 (M+H)⁺.

### Example 78: {4-[4-Bromo-phenyl)-phenyl-amino]-4'-methyl-[1,4']bipiperidinyl-1'-yl}-(2,4-dimethyl-1-oxy-pyridin-3-yl)-methanone

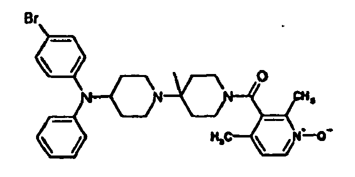

It is prepared from (4-bromo-phenyl)-phenyl-piperidin-4-yl-amine and 4-phenylamino-piperidine-1-carboxylic tert.-butyl ester using a procedure as described in example 1, 1c) and 1d). MS/ESI 577 (M+H)⁺

### Example 79: [4-(Benzo[1,3]dioxol-5-yl-benryl-amino)-4'-methyl-[1,4']bipiperidinyl-1'-yl]-(2,6-dimethyl-phenyl)methanone

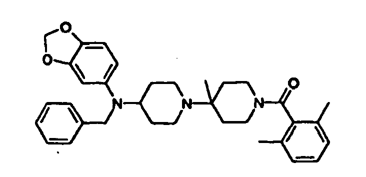

It is prepared from 1'-(2,6-dimethyl-benzoyl)-4'-methyl-[1,4']bipiperidinyl-4-one and benzo[1,3]dioxol-5-ylamine by following a procedure as described in examples 51 and 52. MS/ESI 540 (M+H)⁺.

### Example 80: {4-[1,3-Benzodioxol-5-yl-(2-methyl-thiazol-4-ylmethyl)-amino]-4'-methyl-1,4'-bipiperidinyl-1'-yl}-(2,6-dimethyl-phenyl)-methanone

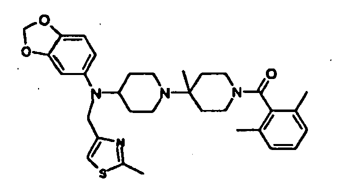

It is prepared from 1'-(2,6-dimethyl-benzoyl)-4'-methyl-[1,4']bipiperidinyl-4-one and benzo[1,3]dioxol-5-ylamine by following a procedure as described in examples 51 and 52.
MS(ESI) 561 (M+H)⁺

### Example 81: {4-[(4-Bromo-phenyl)-pyridin-3-yl-amino]-4'-methyl-[1,4']bipiperidinyl-1'-yl}-(2,4-dimethyl-1-oxy-pyridin-3-yl)-methanone

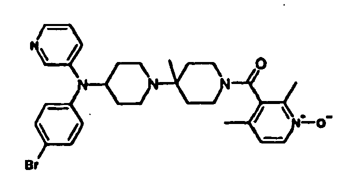

It is prepared from 4-(pyridin-3-ylamino)-piperidine-1-carboxyllc acid tert-butyl ester and 1,4-dibromo-benzene by following a procedure as described in example 1. MS/ESI 578 (M+H)⁺.

### Example 82: [4-(Benzyl-phenyl-amino)-4'-methyl-[1,4']bipiperidinyl-1'-yl]-(2,4-dimethyl-1-oxy-pyridin-3-yl)-methanone

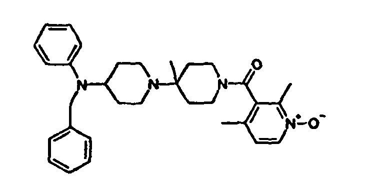

It is prepared from phenyl-piperidin-4-yl-amine by following a procedure as described in examples 52 and 1. MS/ESI 513 (M+H)⁺.

### Example 83: (2,4-Dimethyl-1-oxy-pyridin-3-yl)-{4'-methyl-4-[(2-methyl-thiazol-4-ylmethyl)-phenyl-amino]-[1,4']bipiperidinyl-1'-yl}-methanone

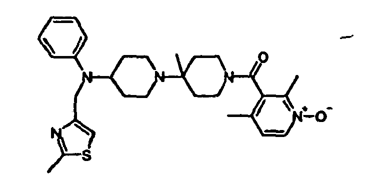

It prepared from (4'-methyl-[1,4']bipiperidinyl-4-yl)-(2-methyl-thlazol-4-ylmethyl)-phenylamine using a procedure as described in example 1. MS/ESI 534 (M+H)⁺.

(4'-methyl-[1,4']bipiperldinyl-4-yl)- (2-methyl-thiazol-4-ylmethyl)-phenyl-amine, used as starting material, is obtained as follows: a mixture of 4-(benzyl-phenylamino)-4'-methyl-[1,4']bipiperidinyl-1'-carboxylic acid tert.-butyl ester (1.0 g, 2.16 mmol), ammonium formate (0.5 g, 7.92 mmol) and Pd(OH)₂ (20%) on charcoal (0.25 g) in methanol (25 ml) is heated under reflux for 3 h. The catalyst is filtered off and washed with methanol. The solvent is removed and the residue dissolved in ethyl acetate. The organic solution is extracted with 1 N NaOH and brine and dried with sodium sulfate. Removal of the solvent gives crude 4'-methyl-4-phenylamino-[1,4']bipiperidinyl-1'-carboxylic acid tert.-butyl ester which is used in the next step without further purification. MS/ESI 374 (M+H)⁺.

4'-Methyl-4-phonylamino-[1,4']bipiperidinyl-1'-carboxylic acid tert.-butyl ester is converted into (4'-methyl-[1,4']bipiperidinyl-4-yl)- (2-methyl-thiazol-4-ylmethyl)-phenyl-amine using a procedure as described in examples 52 and 1d).

The compounds of formula I in free form or in pharmaceutically acceptable salt form exhibit valuable pharmacological properties, e.g. as CCR5 antagonists, e.g. as indicated in in vitro tests and therefore indicated for therapy.

### a) CCR5 membrane binding assay

Human CCR5 is used to generate stable transfectants in CHO K1 cells. Membranes prepared from these CCR5 transfectants are used in a radioligand binding assay using 125-I MIP-1α as a ligand and the compounds of formula I are tested for inhibitory activity. The data are reported as IC₅₀, i.e. the concentration of compound required to achieve 50% inhibition of [1-125]MIP-1 α binding. In this assay, compounds of formula I have an IC₅₀ ≤ 1µM. Compounds of Examples 16, 53 and 83 have an IC₅₀ of 2 to 3 nM, respectively.

### b) CCR5 functional assay- Ca²⁺ mobilization

Human CCR5 is used to generate stable transfectants in CHO K1 cells. These CCR5 transfectants are used for assessing Ca²⁺ mobilization in response to stimulation by the CCR5 ligands MIP-1α, MIP-1β, HCC-1(9-74) or RANTES. For the assay the cells are loaded with a Ca²⁺-sensitive fluorochrome (Fluo3 or Fluo4). Ligand concentrations between 0.01 - 100 nM are used to induce Ca²⁺ mobilization which is monitored in a fluorometer with appropriate settings.

To assess the activity of the compounds to be tested, a baseline fluorescence reading is taken after which the compounds at the desired concentration are added to the cells and fluorescence is further recorded for a certain time to assess whether compounds show agonistic effects. Next the agonist is added to the mixture and fluorescence monitored. The inhibition of Ca²⁺ flux in the presence of the compounds to be tested is calculated from the inhibition of maximal fluorescence induced by the agonist. IC₅₀ values are calculated from dose-response curves obtained with the compounds. In this assay, compounds of formula I have an IC₅₀ ≤ 1µM. For example, compounds of Example 1, 18 and 52 have an IC₅₀ of 10, 9 and 4, respectively.

### c) CCR5 functional assay - chemotaxis

CCR5 transfectants are generated in Jurkat T cells or the mouse pre B cell line L1.2.

Migration of CCR5 transfectants is tested in transwell tissue chamber inserts system with the CCR5 agonist MIP-1α at concentrations of 1-100 nM. Cells migrated in response to the agonist compared to a buffer control are quantified in a flow cytometer. The compounds to be tested are added to the cells and the agonist compartments. IC₅₀ values are calculated from concentration-response curves obtained with the compounds in the presence of MIP-1α. In this assay, compounds of formula I have an IC₅₀ ≤ 1µM.

### d) Experiments performed in murine animal models show that vessel wall remodeling after experimental injury (e.g. induced by allotransplantation) is significantly inhibited in the absence of functional CCR5.

The compounds of formula I are, therefore, useful in the prevention and/or treatment of diseases or disorders mediated by interactions between chemokine receptors, e.g. CCR5, and their ligands, e.g. in transplantation, such as acute or chronic rejection of organ, tissue or cell allo- or xenografts or delayed graft function, autoimmune diseases, e.g. rheumatoid arthritis, systemic lupus erythematosus, Hashimoto's thyroidis, multiple sclerosis, myasthenia gravis, diabetes type I or II and the disorders associated therewith, vasculitis, pernicious anemia, Sjoegren syndrome, uveitis, psoriasis, alopecia areata and others, allergic diseases, e.g. allergic asthma, atopic dermatitis, allergic rhinitis/conjunctivitis, allergic contact dermatitis, inflammatory diseases optionally with underlying aberrant reactions, e.g. inflammatory bowel disease, Crohn's disease or ulcerative colitis, intrinsic asthma, inflammatory lung injury, inflammatory liver injury, inflammatory glomerular injury, atherosclerosis, osteoarthritis, irritant contact dermatitis and further eczematous dermatitises, seborrhoeic dermatitis, cutaneous manifestations of immunologically-mediated disorders, inflammatory eye disease, keratoconjunctivitis, myocarditis or hepatitis, ischemia/reperfusion injury, e.g. myocardial infarction, stroke, gut ischemia, renal failure or hemorrhage shock, traumatic shock and others, cancer, e.g. solid tumors or lymphatic cancer such as T cell lymphomas or T cell leukemias, metastasizing or angiogenesis, infectious diseases, e.g. toxic shock (e.g. superantigen induced), septic shock, adult respiratory distress syndrome or viral infections, e.g. AIDS. By transplantation is meant allo- or xeno grafts of e.g. cells, tissues or solid organs, for example pancreatic islets, stem cells, bone marrow, corneal tissue, neuronal tissue, heart, lung, combined heart-lung, kidney, liver, bowel, pabcreas, trachea or oesophagus. Chronic rejection is also named graft vessel diseases.

For the above uses the required dosage will of course vary depending on the mode of administration, the particular condition to be treated and the effect desired. In general, satisfactory results are indicated to be obtained systemically at daily dosages of from about 0.01 to 10 mg/kg per body weight. An indicated daily dosage in the larger mammal, e.g. humans, is in the range from about 0.5 mg to about 1000 mg, conveniently administered, for example, in divided doses up to four times a day or in retard form. Suitable unit dosage forms for oral administration comprise from ca. 1 to 500 mg active ingredient.

The compounds of formula I may be administered by any conventional route, in particular enterally, e.g. orally, e.g. in the form of tablets or capsules, or parenterally, e.g. in the form of injectable solutions or suspensions, topically, e.g. in the form of lotions, gels, ointments or creams, or in a nasal or a suppository form. Pharmaceutical compositions comprising a compound of formula I in free form or in pharmaceutically acceptable salt form in association with at least one pharmaceutical acceptable carrier or diluent may be manufactured in conventional manner by mixing with a pharmaceutically acceptable carrier or diluent.

The compounds of formula I may be administered in free form or in pharmaceutically acceptable salt form e.g. as indicated above. Such salts may be prepared in conventional manner and exhibit the same order of activity as the free compounds.

In accordance with the foregoing also described are:
1.1 A method for preventing or treating disorders or diseases mediated by interactions between chemokine receptors and their ligands, e.g. such as indicated above, in a subject in need of such treatment, which method comprises administering to said subject an effective amount of a compound of formula I or a pharmaceutically acceptable salt thereof;
1.2 A method for preventing or treating acute or chronic transplant rejection or inflammatory or autoimmune diseases, e.g. as indicated above, in a subject in need of such treatment, which method comprises administering to said subject an effective amount of a compound of formula I or a pharmaceutically acceptable salt thereof;

The present invention further provides
2. A compound of formula I or a pharmaceutically acceptable salt thereof for use as a pharmaceutical, e.g. in any of the methods as indicated under 1.1 or 1.2 above.
3. A pharmaceutical composition, e.g. for use in any of the methods as in 1.1 or 1.2 above comprising a compound of formula I or a pharmaceutically acceptable salt thereof in association with a pharmaceutically acceptable diluent or carrier therefor.
4. A compound of formula I or a pharmaceutically acceptable salt thereof for use in the preparation of a pharmaceutical composition for use in any of the method as in 1.1 or 1.2 above.

The compounds of formula I may be administered as the sole active ingredient or in conjunction with, e.g. as an adjuvant to, other drugs e.g. in immunosuppressive or immunomodulating regimens or other anti-inflammatory agents, e.g. for the treatment or prevention of allo- or xenograft acute or chronic rejection or inflammatory or autoimmune disorders, a chemotherapeutic agent or an anti-infective agent, e.g. an anti-viral agent such as e.g. an anti-retroviral agent or an antibiotic. For example, the compounds of formula I may be used in combination with a calcineurin inhibitor, e.g. cyclosporin A or FK 506; a macrocyclic lactone having immunosuppressive properties, e.g. rapamycin, 40-O-(2-hydroxyethyl)-rapamycin, CC1779 or ABT578; an ascomycin having immunosuppressive properties, e.g. ABT-281, ASM981, etc.; corticosteroids; cyclophosphamide; azathioprine; methotrexate; leflunomide; mizoribine; mycophenolic acid; mycophenolate mofetil; 15-deoxyspergualine or an immunosuppressive homologue, analogue or derivative thereof; an accelerating lymphocyte homing agent, e.g. FTY720; monoclonal antibodies to leukocyte receptors, e.g., MHC, CD2, CD3, CD4, CD7, CD8, CD11a/CD18, CD25, CD27, CD28, CD40. CD45, CD58, CD80, CD86, CD137, ICOS, CD150 (SLAM), OX40, 4-1 BB or to their ligands, e.g. CD154, or antagonists thereof; other immunomodulatory compounds, e.g. a recombinant binding molecule having at least a portion of the extracellular domain of CTLA4 or a mutant thereof, e.g. an at least extracellular portion of CTLA4 or a mutant thereof joined to a non-CTLA4 protein sequence, e.g. CTLA4lg (for ex. designated ATCC 68629) or a mutant thereof, e.g. LEA29Y ; adhesion molecule inhibitors, e.g. LFA-1 antagonists, ICAM-1 or -3 antagonists, VCAM-4 antagonists or VLA-4 antagonists; or antichemokine antibodies or antichemokine receptor antibodies or low molecular weight chemokine receptor antagonists, e.g. anti MCP-1 antibodies.

Where the compounds of formula I are administered in conjunction with other immunosuppressive / immunomodulatory, anti-inflammatory or chemotherapeutic therapy, dosages of the co-administered immunosuppressant, immunomodulatory, anti-inflammatory or chemotherapeutic compound will of course vary depending on the type of co-drug employed, e.g. whether it is a steroid or a calcineurin inhibitor, on the specific drug employed, on the condition being treated and so forth. In accordance with the foregoing also described is:
5. A method as defined above comprising co-administration, e.g. concomitantly or in sequence, of a therapeutically effective non-toxic amount of a compound of formula I and at least a second drug substance, e.g. an immunosuppressant, immunomodulatory, anti-inflammatory, anti-infective or chemotherapeutic drug, e.g. as indicated above. The present invention provides in a yet further aspect
6. A pharmaceutical combination, e.g. a kit, comprising a) a first agent which is a CCR5 antagonist, e.g. a compound of formula I as disclosed herein, in free form or in pharmaceutically acceptable salt form, and b) at least one co-agent, e.g. an immunosuppressant, immunomodulatory, anti-inflammatory, anti-infective or chemotherapeutic drug. The kit may comprise instructions for its administration.

The terms "co-administration" or "combined administration" or the like as utilized herein are meant to encompass administration of the selected therapeutic agents to a single patient, and are intended to include treatment regimens in which the agents are not necessarily administered by the same route of administration or at the same time.

The term "pharmaceutical combination" as used herein means a product that results from the mixing or combining of more than one active ingredient and includes both fixed and non-fixed combinations of the active ingredients. The term "fixed combination" means that the active ingredients, e.g. a compound of formula I and a co-agent, are both administered to a patient simultaneously in the form of a single entity or dosage. The term "non-fixed combination" means that the active ingredients, e.g. a compound of formula I and a co-agent, are both administered to a patient as separate entities either simultaneously, concurrently or sequentially with no specific time limits, wherein such administration provides therapeutically effective levels of the 2 compounds in the body of the patient. The latter also applies to cocktail therapy, e.g. the administration of 3 or more active ingredients.

## Claims

1. A compound of formula 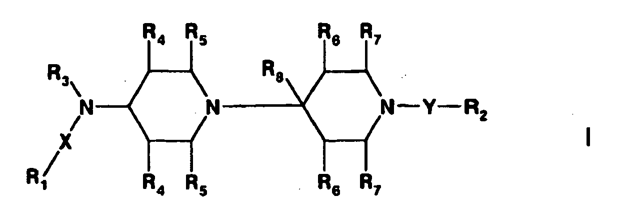 wherein
X is a direct bond; -CH₂-; -CH₂-CH₂-; -CHR₉-; -C(O)-; -O**-;** -NH- or NR₉;
R₁ is optionally R₁₀ and/or R₁₁-substituted phenyl; optionally R₁₀ and/or R₁₁-substituted heteroaryl; optionally R₁₀ and/or R₁₁-substituted heteroaryl N-oxide; or optionally R₁₀ and/or R₁₁-substituted naphthyl;
R₂ has one of the significances given for R₁;or is optionally R₁₀ and/or R₁₁-substituted fluorenyl; optionally R₁₀-substituted C₁-C₆ alkyl; optionally R₁₀-substituted C₂-C₆ alkenyl; optionally R₁₀-substituted C₃-C₆ cycloalkyl; optionally R₁₀-substituted adamantyl; or optionally R₁₀₋substituted C₄-C₈ cycloalkenyl;
R₃ has one of the significances given for R₁; or is optionally R₁₀ and/or R₁₁-substituted fluorenyl;
or 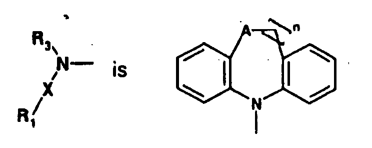 wherein A is -CH₂-, -NH-, -NR₉-, -S-, -SO-, SO₂- or -O-, n is 0, 1 or 2, and the aromatic rings are each, independently optionally R₁₀-substituted;
each of R4, independently, has one of the significances of R₅; or is CN; OH; OR₉; F; Cl;Br; or I;
each of R₅, independently, is H; C₁-C₆ alkyl; C₁-C₆ hydroxyalkyl; C₂-C₆ alkoxyalkyl; C₁-C₆ halogenoalkyl; phenyl; benzyl; or heteroaryl;
each of R₆, independently, has one of the significances given for R₄;
each of R₇, independently, has one of the significances given for R₅;
R₈ is H; C₁-C₆ alkyl; C₂-C₆ alkenyl; C₂-C₆ alkynyl; phenyl; benzyl; CN; CH₂NH₂; CH₂NHR₉; CH₂NR₉R₉; CH₂NHC(O)R₉; CH₂NR₉C(O)R₉; CH₂NHC(O)NHR₉; CH₂NR₉C(O)NHR₉; CH₂NR₉C(O)NR₉R₉; CH₂NHC(O)OR₉; CH₂NR₉C(O)OR₉; CH₂NHSO₂R₉; CH₂N(SO₂R₉)₂; or CH₂NR₉SO₂R₉;
each R₉, independently, is C₁-C₆ alkyl; C₃-C₆ cycloalkyl; C₂-C₆ alkenyl; C₂-C₆ alkynyl; phenyl; benzyl; heteroaryl; or CF₃;
R₁₀ represents 1 to 4 substituents independently selected from C₁-C₆ alkyl; C₁-C₆ hydroxyalkyl; C₂-C₆ alkoxyalkyl; C₁-C₆ halogenoalkyl; C₃-C₆ cycloalkyl; C₂-C₆ alkenyl; C₃-C₆ cycloalkenyl; C₂-C₆ alkynyl; phenyl; heteroaryl; heteroaryl N-oxide ; F; Cl; Br; I; OH; OR₉; CONH₂; CONHR₉; CONR₉R₉ ;OC(O)R₉; OC(O)OR₉; OC(O)NHR₉; OC(O)NR₉R₉; OSO₂R₉; COOH; COOR₉; CF₃; CHF₂; CH₂F; CN; NO₂; NH₂; NHR₉; NR₉R₉; NHC(O)R₉; NR₉C(O)R₉; NHC(O)NHR₉; NHC(O)NH₂; NRgC(O)NHR₉; NR₉C(O)NR₉R₉; NHC(O)OR₉; NR₉C(O)OR₉; NHSO₂R₉; N(SO₂R₉)₂; NR₉SO₂R₉; SR₉; S(O)R₉; SO₂R₉; Si(CH₃)₃ and B(OC(CH₃)₂)₂;
R₁₁ represents two adjacent substituents which form an annulated 4-7 membered nonaromatic ring optionally containing up to two heteroatoms selected independently from N, O and S; and Y is -C(O)-;
in free form or in salt form;
where heteroaryl where mentioned refers to furyl, thienyl, pyrrolyl, imidazolyl, pyrazolyl, thiazolyl, isothiazolyl, oxazolyl, isoxazolyl, oxadiazolyl, thiadiazolyl, triazolyl, tetrazolyl, pyridyl, pyridazinyl, pyrimidinyl, pyrazinyl, triazinyl, tetrazinyl, indolyl, benzothiophenyl, benzofuranyl, benzimidazolyl, indazolyl, benzotriazolyl, benzothiazolyl, .benzoxazolyl, quinolinyl, isoquinolinyl, phthalazinyl, quinoxalinyl, quinazolinyl, cinnolinyl or naphthyridinyl.

2. A compound according to claim 1, wherein R₁ is phenyl or heteroaryl, each being optionally substituted by R₁₀; or phenyl optionally substituted by R₁₁; wherein R₁₀ represents 1 to 3 substituents independently selected from C₁-C₆ alkyl; C₁-C₆ hydroxyalkyl; C₂-C₆ alkoxyalkyl; C₁-C₆ halogenoalkyl; C₃-C₆ cycloalkyl; C₂-C₆ alkenyl; C₃-C₆ cycloalkenyl; C₂-C₆ alkynyl; phenyl; heteroaryl; heteroaryl N-oxide; F; Cl; Br; I; OH; OR₉; CONH₂; CONHR₉; CONR₉R₉; OC(O)R₉; OC(O)OR₉; OC(O)NHR₉; OC(O)NR₉R₉; OSO₂R₉; COOH; COOR₉; CF₃; CHF₂; CH₂F; CN; NO₂; NH₂; NHR₉; NR₉R₉; NHC(O)R₉; NR₉C(O)R₉; NHC(O)NHR₉; NHC(O)NH₂; NR₉C(O)NHR₉; NR₉C(O)NR₉R₉; NHC(O)OR₉; NR₉C(O)OR₉; NHSO₂R₉; N(SO₂R₉)₂; NR₉SO₂R₉; SR₉; S(O)R₉; SO₂R₉ and Si(CH₃)₃ and R₁₁ is an annulated 5 or 6 membered non aromatic ring optionally containing 1 or 2 oxygen atoms, and attached to 2 adjacent carbon atoms;
where heteroaryl where mentioned refers to furyl, thienyl, pyrrolyl, imidazolyl, pyrazolyl, thiazolyl, isothiazolyl, oxazolyl, isoxazolyl, oxadiazolyl, thiadiazolyl, triazolyl, tetrazolyl, pyridyl, pyridazinyl, pyrimidinyl, pyrazinyl, triazinyl, tetrazinyl, indolyl, benzothiophenyl, benzofuranyl, benzimidazolyl, indazolyl, benzotriazolyl, benzothiazolyl, .benzoxazolyl, quinolinyl, isoquinolinyl, phthalazinyl, quinoxalinyl, quinazolinyl, cinnolinyl or naphthyridinyl.

3. A compound according to claim 1, wherein each of R₄, R₅, R₆ or R₇ independently, is H; C₁₋₆ alkyl; or benzyl.

4. A compound according to claim 1, wherein R₈ is H; C₁₋₆ alkyl; or C₂₋₆ alkenyl.

5. A compound according to claim 1 wherein X is a direct bond or -CH₂-.

6. A process for the preparation of a compound of formula I according to claim 1, which process comprises
a) for the preparation of a compound of formula I wherein X is a direct bond, -CH₂-, -CH₂-CH₂- or -CHR₉- and Y is -CO-,
amidating a compound of formula II 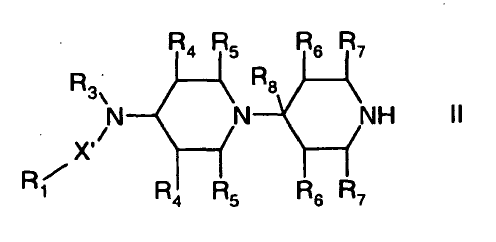 wherein R₁ and R₃ to R₈ are as indicated in claim 1 and X' is a direct bond, -CH₂-, -CH₂-CH₂- or -CHR₉-
with a compound of formula III
R₂-Y'-A' III
wherein R₂ is as defined above, Y' is -CO- and A' is a leaving group, e.g. Cl, Br or OH;
or
b) for the preparation of a compound of formula I wherein X is CH₂-, -CH₂-CH₂- or -CHR₉- and Y is -CO-,
reacting a compound of formula IV 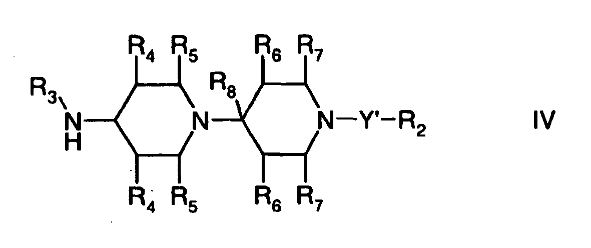 wherein R₂ to R₈ and Y' are as defined above, with a compound of formula V
R₁X"-Hal V
wherein R₁ is as defined above and X" is CH₂- or -CHR₉-;
and, where required, converting the resulting compound of formula I obtained in free form into the desired salt form, or vice versa.

7. A compound according to any one of claims 1 to 5 or a pharmaceutically acceptable salt thereof for use as a pharmaceutical.

8. A pharmaceutical composition comprising a compound of formula I according to claim 1 or a pharmaceutically acceptable salt thereof in association with a pharmaceutically acceptable diluent a carrier therefor.

9. A pharmaceutical combination comprising
a) a first agent which is a compound of formula I according to claim 1, or a pharmaceutically acceptable salt thereof, and
b) at least one co-agent.

10. Use of a compound according to any one of the preceding claims in the manufacture of a medicament for preventing or treating disorders or diseases selected from transplantation, such as acute or chronic rejection of organ, tissue or cell allo- or xenografts or delayed graft function, autoimmune diseases, e.g. rheumatoid arthritis, systemic lupus erythematosus, Hashimoto's thyroidis, multiple sclerosis, myasthenia gravis, diabetes type I or II and the disorders associated therewith, vasculitis, pernicious anemia, Sjoegren syndrome, uveitis, psoriasis, alopecia areata and others, allergic diseases , e.g. allergic asthma, atopic dermatitis, allergic rhinitis/conjunctivitis, allergic contact dermatitis, inflammatory diseases optionally with underlying aberrant reactions, e.g. inflammatory bowel disease, Crohn's disease or ulcerative colitis, intrinsic asthma, inflammatory lung injury, inflammatory liver injury, inflammatory glomerular injury, atherosclerosis, osteoarthritis, irritant contact dermatitis and further eczematous dermatitises, seborrhoeic dermatitis, cutaneous manifestations of immunologically-mediated disorders, inflammatory eye disease, keratoconjunctivitis, myocarditis or hepatitis, ischemia/reperfusion injury, e.g. myocardial infarction, stroke, gut ischemia, renal failure or hemorrhage shock, traumatic shock and others, cancer, e.g. solid tumors or lymphatic cancer such as T cell lymphomas or T cell leukemias, metastasizing or angiogenesis, infectious diseases, e.g. toxic shock (e.g. superantigen induced), septic shock, adult respiratory distress syndrome or viral infections, e.g. AIDS.

11. Use of claim 10 wherein said prevention or treatment are acute or chronic transplant rejection, or inflammatory or autoimmune diseases.

12. Compound according to claim 1 of the formula 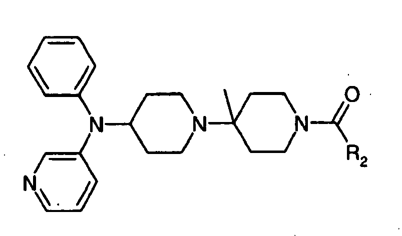 wherein R₂ is

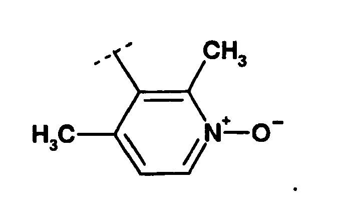

## Patentansprüche

1. Verbindung der Formel I 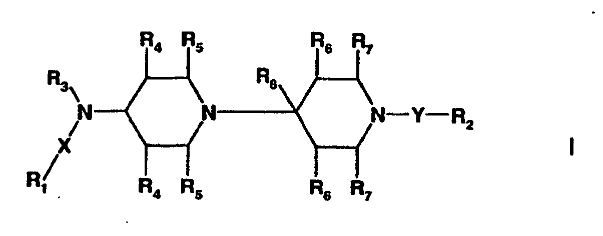 wobei:
X eine direkte Bindung;-CH₂-; -CH₂-CH₂-; -CHR₉-; -C(O)-; -O- ; -NH- oder NR₉ ist;
R₁ gegebenenfalls R₁₀- und/oder R₁₁-substituiertes Phenyl; gegebenenfalls R₁₀- und/oder R₁₁-substituiertes Heteroaryl; gegebenenfalls R₁₀- und/oder R₁₁-substituiertes Heteroaryl-N-oxid; oder gegebenenfalls R₁₀- und/oder R₁₁-substituiertes Naphthyl ist;
R₂ eine der für R₁ angegebenen Bedeutungen hat; oder gegebenenfalls R₁₀- und/oder R₁₁-substituiertes Fluorenyl; gegebenenfalls R₁₀-substituiertes C₁-C₆-Alkyl; gegebenenfalls R₁₀-substituiertes C₂-C₆-Alkenyl; gegebenenfalls R₁₀-substituiertes C₃-C₆-Cycloalkyl; gegebenenfalls R₁₀-substituiertes Adamantyl; oder gegebenenfalls R₁₀-substituiertes C₄-C₈-Cycloalkenyl ist; R₃ eine der für R₁ angegebenen Bedeutungen hat; oder gegebenenfalls R₁₀- und/oder R₁₁-substituiertes Fluorenyl ist; oder 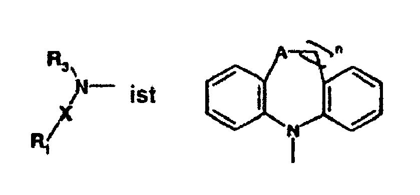 wobei:
A für -CH₂-, -NH-, -NR₉-, -S-, -SO-, SO₂- oder -O- steht, n gleich 0, 1 oder 2 ist, und die aromatischen Ringe jeweils gegebenenfalls unabhängig voneinander R₁₀-substituiert sind;
R₄ jeweils unabhängig eine der Bedeutungen für R₅ haben; oder CN; OH; OR₉; F; Cl; Br; oder I ist;
R₅ jeweils unabhängig H; C₁-C₆-Alkyl; C₁-C₆-Hydroxyalkyl; C₂-C₆-Alkoxyalkyl; C₁-C₆-Halogenalkyl; Phenyl; Benzyl; oder Heteroaryl ist;
R₆ jeweils unabhängig eine der für R₄ gegebenen Bedeutungen hat;
R₇ jeweils unabhängig eine der für R₅ gegebenen Bedeutungen hat;
R₈ für H; C₁-C₆-Alkyl; C₂-C₆-Alkenyl; C₂-C₆-Alkinyl; Phenyl; Benzyl; CN; CH₂NH₂; CH₂NHR₉; CH₂NR₉R₉; CH₂NHC(O)R₉; CH₂NR₉C(O)R₉; CH₂NHC(O)NHR₉; CH₂NR₉C(O)NHR₉; CH₂NR₉C(O)NR₉R₉; CH₂NHC(O)OR₉; CH₂NR₉C(O)OR₉; CH₂NHSO₂R₉; CH₂N(SO₂R₉)₂; oder CH₂NR₉SO₂R₉ steht;
R₉ jeweils unabhängig für C₁-C₆-Alkyl; C₃-C₆-Cycloalkyl; C₂-C₆-Alkenyl; C₂-C₆-Alkinyl; Phenyl; Benzyl; Heteroaryl; oder CF₃ steht;
R₁₀ 1 bis 4 Substituenten veranschaulicht, die unabhängig ausgewählt sind aus C₁-C₆-Alkyl; C₁-C₆-Hydroxyalkyl; C₂-C₆-Alkoxyalkyl; C₁-C₆-Halogenoalkyl; C₃-C₆-Cycloalkyl; C₂-C₆-Alkenyl; C₃-C₆-Cycloalkenyl; C₂-C₆-Alkinyl; Phenyl; Heteroaryl; Heteroaryl-N-oxid; F; Cl; Br; I; OH; OR₉; CONH₂; CONHR₉; CONR₉R₉; OC(O)R₉; OC(O)OR₉; OC(O)NHR₉; OC(O)NR₉R₉; OSO₂R₉; COOH; COOR₉; CF₃; CHF₂; CH₂F; CN; NO₂; NH₂; NHR₉; NR₉R₉; NHC(O)R₉; NR₉C(O)R₉; NHC(O)NHR₉; NHC(O)NH₂; NR₉C(O)NHR₉; NR₉C(O)NR₉R₉; NHC(O)OR₉; NR₉C(O)OR₉; NHSO₂R₉; N(SO₂R₉)₂; NR₉SO₂R₉; SR₉; S(O)R₉; SO₂R₉; Si(CH₃)₃ und B(OC(CH₃)₂)₂;
R₁₁ zwei benachbarte Substituenten veranschaulicht, die einen annelierten 4- bis 7-gliedrigen nichtaromatischen Ring bilden, der gegebenenfalls bis zu zwei Heteroatome trägt, die unabhängig ausgewählt sind aus N, O und S; und
Y -C(O)- ist
in freier Form oder in Salzform.
wobei das Heteroaryl, wenn es erwähnt ist, für Furyl, Thienyl, Pyrrolyl, Imidazolyl, Pyrazolyl, Thiazolyl, Isothiazolyl, Oxazolyl, Isoxazolyl, Oxadiazolyl, Thiadiazolyl, Triazolyl, Tetrazolyl, Pyridyl, Pyridazinyl, Pyrimidinyl, Pyrazinyl, Triazinyl, Tetrazinyl, Indolyl, Benzothiophenyl, Benzofuranyl, Benzimidazolyl, Indazolyl, Benzotriazolyl, Benzothiazolyl, Benzoxazolyl, Chinolinyl, Isochinolinyl, Phthalazinyl, Chinoxalinyl, Chinazolinyl, Cinnolinyl oder Naphthyridinyl steht.

2. Verbindung nach Anspruch 1, wobei R₁ für Phenyl oder Heteroaryl steht, die jeweils gegebenenfalls durch R₁₀ substituiert sind; oder Phenyl, das gegebenenfalls durch R₁₁ substituiert ist, wobei R₁₀ 1 bis 3 Substituenten veranschaulicht, die unabhängig voneinander ausgewählt sind aus C₁-C₆-Alkyl; C₁-C₆-Hydroxyalkyl; C₂-C₆-Alkoxyalkyl; C₁-C₆-Halogenoalkyl; C₃-C₆-Cycloalkyl; C₂-C₆-Alkenyl; C₃-C₆-Cycloalkenyl; C₂-C₆-Alkinyl; Phenyl; Heteroaryl; Heteroaryl-N-oxid; F; Cl; Br; I; OH; OR₉; CONH₂; CONHR₉; CONR₉R₉; OC(O)R₉; OC(O)OR₉; OC(O)NHR₉; OC(O)NR₉R₉; OSO₂R₉; COOH; COOR₉; CF₃; CHF₂; CH₂F; CN; NO₂; NH₂; NHR₉; NR₉R₉; NHC(O)R₉; NR₉C(O)R₉; NHC(O)NHR₉; NHC(O)NH₂; NR₉C(O)NHR₉; NR₉C(O)NR₉R₉; NHC(O)OR₉; NR₉C(O)OR₉; NHSO₂R₉; N(SO₂R₉)₂; NR₉SO₂R₉; SR₉; S(O)R₉; SO₂R₉; und Si(CH₃)₃ und R₁₁ ein annelierter 5- oder 6-gliedriger nichtaromatischer Ring ist, der gegebenenfalls 1 oder 2 Sauerstoffatome enthält und an 2 benachbarte Kohlenstoffatome gebunden ist;
wobei das Heteroaryl, wenn es erwähnt ist, für Furyl, Thienyl, Pyrrolyl, Imidazolyl, Pyrazolyl, Thiazolyl, Isothiazolyl, Oxazolyl, Isoxazolyl, Oxadiazolyl, Thiadiazolyl, Triazolyl, Tetrazolyl, Pyridyl, Pyridazinyl, Pyrimidinyl, Pyrazinyl, Triazinyl, Tetrazinyl, Indolyl, Benzothiophenyl, Benzofuranyl, Benzimidazolyl, Indazolyl, Benzotriazolyl, Benzothiazolyl, Benzoxazolyl, Chinolinyl, Isochinolinyl, Phthalazinyl, Chinoxalinyl, Chinazolinyl, Cinnolinyl oder Naphthyridinyl steht.

3. Verbindung nach Anspruch 1, wobei die Reste R₄, R₅, R₆ oder R₇ jeweils unabhängig voneinander H, C₁₋₆-Alkyl oder Benzyl sind.

4. Verbindung nach Anspruch 1, wobei R₈ für H, C₁₋₆-Alkyl oder C₂₋₆-Alkenyl steht.

5. Verbindung nach Anspruch 1, wobei X für eine direkte Bindung oder -CH₂- steht.

6. Verfahren zur Herstellung einer Verbindung der Formel I nach Anspruch 1, wobei das Verfahren umfasst:
a) zur Herstellung einer Verbindung der Formel I, wobei X für eine direkte Bindung, -CH₂-, -CH₂-CH₂- oder -CHR₉- ist und Y für -CO- steht,
Amidieren einer Verbindung der Formel II 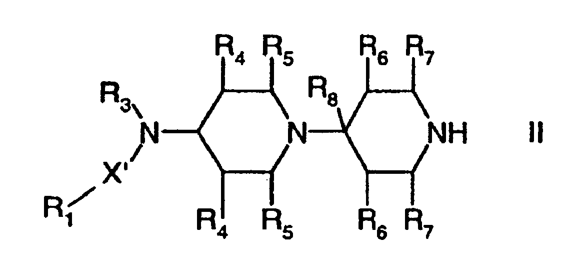 wobei R₁ and R₃ bis R₈ wie in Anspruch 1 angegeben sind and X' für eine direkte Bindung, -CH₂-, -CH₂-CH₂- oder -CHR₉- steht,
mit einer Verbindung der Formel III
R₂-Y'-A' III
wobei R₂ die vorstehend definierte Bedeutung hat, Y' für -CO- steht and A' eine Abgangsgruppe, beispielsweise Cl oder Br oder OH ist,
b) zur Herstellung einer Verbindung der Formel I, wobei X für CH₂-, -CH₂-CH₂- oder -CHR₉- steht und Y für -COsteht, Umsetzen einer Verbindung der Formel IV 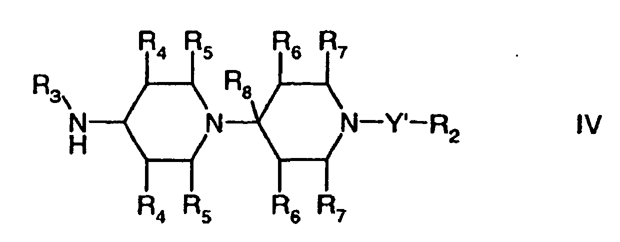 wobei R₂ bis R₈ und Y' die vorstehend definierte Bedeutung haben, mit einer Verbindung der Formel V
R₁-X"-Hal V
wobei R₁ die vorstehend definierte Bedeutung hat and X" für CH₂- oder -CHR₉- steht;
und bei Bedarf Umwandeln der resultierenden Verbindung der Formel I, die in freier Form erhalten wird, in die gewünschte Salzform, oder umgekehrt.

7. Verbindung nach einem der Ansprüche 1 bis 5 oder ein pharmazeutisch unbedenkliches Salz davon zur Verwendung als Pharmazeutikum.

8. Pharmazeutische Zusammensetzung, umfassend eine Verbindung der Formel I nach Anspruch 1 oder ein pharmazeutisch unbedenkliches Salz davon zusammen mit einem pharmazeutisch unbedenklichen Verdünnungsmittel oder Träger dafür.

9. Pharmazeutische Kombination, umfassend
a) ein erstes Mittel, bei dem es sich um eine Verbindung der Formel I nach Anspruch 1 oder um ein pharmazeutisch unbedenkliches Salz davon handelt, und
b) wenigstens ein gemeinsam verabreichtes Mittel.

10. Verwendung einer Verbindung nach einem der vorhergehenden Ansprüche zur Herstellung eines Arzneistoffs zur Prävention oder Behandlung von Beschwerden oder Krankheiten, ausgewählt aus Transplantation, wie von akuter und chronischer Abstoßung von Organ-, Gewebe- oder Zellallo- oder - xenotransplantaten oder verzögerter Transplantatfunktion, Autoimmunkrankheiten, z.B. rheumatoider Arthritis, systemischem Lupus erythematodes, Hashimoto-Thyreoiditis, Multipler Sklerose, Myasthenia gravis, Diabetes vom Typ I oder II und den damit einhergehenden Erkrankungen, Vaskulitis, perniziöser Anämie, Sjoegren-Syndrom, Uveitis, Psoriasis, Alopecia areata sowie anderen, allergischen Krankheiten, z.B. allergischem Asthma, atopischer Dermatitis, allergischer Rhinitis/Konjunktivitis, allergischer Kontaktdermatitis, entzündlichen Krankheiten gegebenenfalls mit zugrundeliegenden aberranten Reaktionen, z.B. entzündlicher Darmerkrankung, Morbus Crohn oder Colitis ulcerosa, intrinsischem Asthma, entzündlicher Lungenverletzung, entzündlicher Leberverletzung, entzündlicher Glomerulumverletzung, Atherosklerose, Osteoarthritis, reizender Kontaktdermatitis und anderen ekzematösen Hautkrankheiten, seborrhoischer Dermatitis, kutanen Manifestationen von über das Immunsystem vermittelten Erkrankungen, entzündlichen Augenkrankheiten, Keratokonjunktivitis, Myokarditis oder Hepatitis, Ischämie/Reperfusionsverletzung, z.B. Herzinfarkt, Schlaganfall, Darmischämie, Nierenversagen oder hämorrhagischem Schock, traumatischem Schock und anderen, Krebs, beispielsweise festen Tumoren oder lymphatischem Krebs, wie T-Zellen-Lymphomen oder T-Zellen-Leukämien, Metastasen oder Angiogenese, Infektionskrankheiten, z.B. toxischem Schock (z.B. superantigeninduziert), septischem Schock, Schocklunge oder Virusinfektionen, z.B. AIDS.

11. Verwendung nach Anspruch 10, wobei die Prävention oder die Behandlung akute oder chronische Transplantatabstoßung oder entzündliche oder Autoimmunerkrankungen betrifft.

12. Verbindung nach Anspruch 1, der Formel 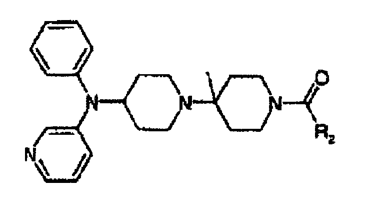 wobei R₂ die Formel 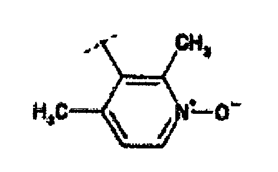 hat.

## Revendications

1. Composé de formule I 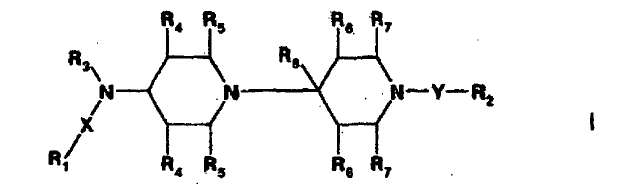 dans laquelle X est une liaison directe ; -CH₂-; -CH₂-CH₂- ; -CHR₉-; -C(O)- ; -O-; -NH- ou NR₉ ;
R₁ est phényle éventuellement substitué par R₁₀ et/ou R₁₁ ; hétéroaryle éventuellement substitué par R₁₀ et/ou R₁₁ ; hétéroaryl-N-oxyde éventuellement substitué par R₁₀ et/ou R₁₁ ; ou naphtyle éventuellement substitué par R₁₀ et/ou R₁₁ ;
R₂ a l'une des significations données pour R₁ ; ou est fluorényle éventuellement substitué par R₁₀ et/ou R₁₁ ; alkyle en C₁-C₆ éventuellement substitué par R₁₀ ; alcényle en C₂-C₆ éventuellement substitué par R₁₀ ; cycloalkyle en C₃-C₆ éventuellement substitué par R₁₀ ; adamantyle éventuellement substitué par R₁₀ ; ou cycloalcényle en C₄-C₈ éventuellement substitué par R₁₀ ;
R₃ a l'une des significations données pour R₁ ; ou est fluorényle éventuellement substitué par R₁₀ et/ou R₁₁ ;
ou 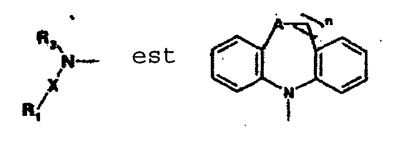 où A est -CH₂-, -NH-, -NR₉-, -S-, -SO-, SO₂-, ou -O-, n est O, 1 ou 2, et les cycles aromatiques sont chacun indépendamment éventuellement substitués par R₁₀ ;
chaque R₄ a indépendamment l'une des significations de R₅ ; ou est CN ; OH ; OR₉ ; F ; Cl ; Br ; ou I ;
chaque R₅ est indépendamment H ; alkyle en C₁-C₆, hydroxyalkyle en C₁-C₆ ; alcoxyalkyle en C₂-C₆ ; halogénoalkyle en C₁-C₆ ; phényle ; benzyle ; ou hétéroaryle ;
chaque R₆ a indépendamment l'une des significations données pour R₄ ;
chaque R₇ a indépendamment l'une des significations données pour R₅ ;
R₈ est H ; alkyle en C₁-C₆ ; alcényle en C₂-C₆ ; alcynyle en C₂-C₆ ; phényle ; benzyle ; CN, CH₂NH₂ ; CH₂NHR₉ ; CH₂NR₉R₉ ; CH₂MHC(O)R₉ ; CH₂NR₉C(O)R₉ ; CH₂NHC(O)NHR₉ ; CH₂NR₉C(O)NHR₉ ; CH₂NR₉C(O)NR₉R₉ ; CH₂NHC(O)OR₉ ; CH₂NR₉C(O)OR₉ ; CH₂NHSO₂R₉ ; CH₂N(SO₂R₉)₂ ou CH₂NR₉SO₂R₉ ;
chaque R₉ est indépendamment alkyle en C₁-C₆ ; cycloalkyle en C₃-C₆ ; alcényle en C₂-C₆ ; alcynyle en C₂-C₆ ; phényle ; benzyle ; hétéroaryle ; ou CF₃ ;
R₁₀ représente de 1 à 4 substituants choisis indépendamment parmi alkyle en C₁-C₆ ; hydroxyalkyle en C₁-C₆ ; alcoxyalkyle en C₂-C₆ ; halogénoalkyle en C₁-C₆ ; cycloalkyle en C₃-C₆ ; alcényle en C₂-C₆ ; cycloalcényle en C₃-C₆ ; alcynyle en C₂-C₆ ; phényle ; hétéroaryle ; hétéroaryl-N-oxyde ; F ; Cl ; Br ; I ; OH ; OR₉ ; CONH₂ ; CONHR₉ ; CONR₉R₉ ; OC(O)R₉ ; OC(O)OR₉ ; OC(O)NHR₉ ; OC(O)NR₉R₉ ; OSO₂R₉ ; COOH ; COOR₉ ; CF₃ ; CHF₂ ; CH₂F ; CN ; NO₂ ; NH₂ ; NHR₉ ; NR₉R₉ ; NHC(O)R₉ ; NR₉C(O)R₉ ; NHC(O)NHR₉ ; NHC(O)NH₂ ; NR₉C(O)NHR₉ ; NR₉C(O)NR₉R₉ ; NHC(O)OR₉ ; NR₉C(O)OR₉ ; NHSO₂R₉ ; N(SO₂R₉)₂ ; NR₉SO₂R₉ ; SR₉ ; S(O)R₉ ; SO₂R₉ ; Si(CH₃)₃ et B(OC(CH₃)₂)₂ ;
R₁₁ représente deux substituants adjacents qui forment un cycle non aromatique condensé à 4-7 chaînons contenant éventuellement jusqu'à deux hétéroatomes choisis indépendamment parmi N, O et S ; et
Y est -C(O)- ;
sous une forme libre ou sous forme de sel ;
où hétéroaryle, lorsqu'il est mentionné, désigne furyle, thiényle, pyrrolyle, imidazolyle, pyrazolyle, thiazolyle, isothiazolyle, oxazolyle, isoxazolyle, oxadiazolyle, thiadiazolyle, triazolyle, tétrazolyle, pyridyle, pyridazinyle, pyrimidinyle, pyrazinyle, triazinyle, tétrazinyle, indolyle, benzothiophényle, benzofuranyle, benzimidazolyle, indazolyle, benzotriazolyle, benzothiazolyle, benzoxazolyle, quinoléinyle, isoquinoléinyle, phtalazinyle, quinoxalinyle, quinazolinyle, cinnolinyle ou naphtyridinyle.

2. Composé selon la revendication 1, **caractérisé en ce que** R₁ est phényle ou hétéroaryle, chacun étant éventuellement substitué par R₁₀ ; ou phényle éventuellement substitué par R₁₁ ; où R₁₀ représente de 1 à 3 substituants choisis indépendamment parmi alkyle en C₁-C₆ ; hydroxyalkyle en C₁-C₆ ; alcoxyalkyle en C₂-C₆ ; halogénoalkyle en C₁-C₆ ; cycloalkyle en C₃-C₆ ; alcényle en C₂-C₆ ; cycloalcényle en C₃-C₆ ; alcynyle en C₂-C₆ ; phényle ; hétéroaryle ; hétéroaryl-N-oxyde ; F ; Cl ; Br ; I ; OH ; OR₉ ; CONH₂ ; CONHR₉ ; CONR₉R₉ ; OC(O)R₉ ; OC(O)OR₉ ; OC(O)NHR₉ ; OC(O)NR₉R₉ ; OSO₂R₉; COOH ; COOR₉ ; CF₃ ; CHF₂ ; CH₂F ; CN ; NO₂ ; NH₂; NHR₉ ; NR₉R₉ ; NHC(O)R₉ ; NR₉C(O)R₉ ; NHC(O)NHR₉ ; NHC(O)NH₂ ; NR₉C(O)NHR₉ ; NR₉C(O)NR₉R₉ ; NHC(O)OR₉ ; NR₉C(O)OR₉ ; NHSO₂R₉ ; N(SO₂R₉)₂ ; NR₉SO₂R₉ ; SR₉ ; S(O)R₉ ; SO₂R₉ et Si(CH₃)₃ et R₁₁ est un cycle non aromatique condensé à 5 ou 6 chaînons contenant éventuellement 1 ou 2 atomes d'oxygène, et lié à 2 atomes de carbone adjacents ;
où hétéroaryle, lorsqu'il est mentionné, désigne furyle, thiényle, pyrrolyle, imidazolyle, pyrazolyle, thiazolyle, isothiazolyle, oxazolyle, isoxazolyle, oxadiazolyle, thiadiazolyle, triazolyle, tétrazolyle, pyridyle, pyridazinyle, pyrimidinyle, pyrazinyle, triazinyle, tétrazinyle, indolyle, benzothiophényle, benzofuranyle, benzimidazolyle, indazolyle, benzotriazolyle, benzothiazolyle, benzoxazolyle, quinoléinyle, isoquinoléinyle, phtalazinyle, quinoxalinyle, quinazolinyle, cinnolinyle ou naphtyridinyle.

3. Composé selon la revendication 1, **caractérisé en ce que** chacun de R₄, R₅, R₆ ou R₇ est indépendamment H ; alkyle en C₁₋₆ ; ou benzyle.

4. Composé selon la revendication 1, **caractérisé en ce que** R₈ est H ; alkyle en C₁₋₆ ; ou alcényle en C₂₋₆.

5. Composé selon la revendication 1, **caractérisé en ce que** X est une liaison directe ou -CH₂-.

6. Procédé de préparation d'un composé de formule I selon la revendication 1, lequel procédé comprend
a) pour la préparation d'un composé de formule I dans laquelle X est une liaison directe, -CH₂-, -CH₂-CH₂- ou -CHR₉- et Y est -CO-,
l'amidation d'un composé de formule II 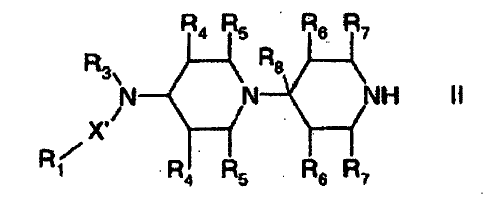 dans laquelle R₁ et R₃ à R₈ sont tels qu'indiqués dans la revendication 1 et X' est une liaison directe ; -CH₂-, -CH₂-CH₂- ou -CHR₉-
avec un composé de formule III
**R₂-Y'-A'** **III**
dans laquelle R₂ est tel que défini ci-dessus, Y' est -CO- et A' est un groupement partant, par exemple Cl, Br ou OH ;
ou
b) pour la préparation d'un composé de formule I dans laquelle X est CH₂-, -CH₂-CH₂- ou -CHR₉- et Y est -CO-,
la réaction d'un composé de formule IV 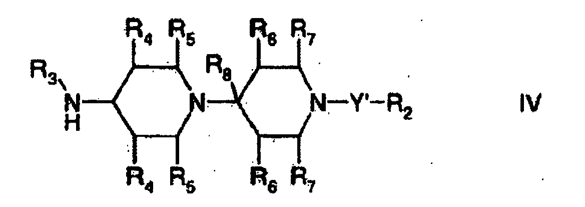 dans laquelle R₂ à R₈ et Y' sont tels que définis ci-dessus, avec un composé de formule V
**R₁-X"-Hal** **V**
dans laquelle R₁ est tel que défini ci-dessus et X'' est CH₂-, ou -CHR₉- ;
et, le cas échéant, la transformation du composé résultant de formule I obtenu sous une forme libre en la forme de sel désirée, ou inversement.

7. Composé selon l'une quelconque des revendications 1 à 5 ou un sel pharmaceutiquement acceptable de celui-ci, destiné à être utilisé comme produit pharmaceutique.

8. Composition pharmaceutique comprenant un composé de formule I selon la revendication 1 ou un sel pharmaceutiquement acceptable de celui-ci en association avec un diluant ou un support pharmaceutiquement acceptable de celui-ci.

9. Combinaison pharmaceutique comprenant
a) un premier agent qui est un composé de formule I selon la revendication 1, ou un sel pharmaceutiquement acceptable de celui-ci, et
b) au moins un co-agent.

10. Utilisation d'un composé selon l'une quelconque des revendications précédentes, dans la fabrication d'un médicament destiné à la prévention ou au traitement d'un trouble ou d'une maladie choisi parmi la transplantation, telle que le rejet aigu ou chronique d'allo- ou xénogreffes d'organes, de tissus ou de cellules ou le fonctionnement retardé des greffons, les maladies auto-immunes, par exemple la polyarthrite rhumatoïde, le lupus érythémateux disséminé, la thyroïdite d'Hashimoto, la sclérose en plaques, la myasthénie grave, le diabète de type I ou II et les troubles associés à celui-ci, la vascularite, l'anémie pernicieuse, le syndrome de Sjoegren, l'uvéite, le psoriasis, l'alopécie en airs et autres, les maladies allergiques, par exemple l'asthme allergique, la dermatite atopique, la rhinite/conjonctivite allergique, la dermatite allergique de contact, les maladies inflammatoires éventuellement avec des réactions aberrantes sous-jacentes, par exemple la maladie inflammatoire de l'intestin, la maladie de Crohn ou la colite ulcéreuse, l'asthme intrinsèque, la lésion inflammatoire des poumons, la lésion inflammatoire du foie, la lésion inflammatoire glomérulaire, l'athérosclérose, l'ostéoarthrite, la dermatite de contact irritante et d'autres dermatites eczémateuses, la dermatite séborrhéique, les manifestations cutanées des troubles médiés immunologiquement, les maladies inflammatoires des yeux, la kératoconjonctivite, la myocardite ou l'hépatite, la lésion d'ischémie/reperfusion, par exemple l'infarctus du myocarde, l'accident vasculaire cérébral, l'ischémie de l'intestin, l'insuffisance rénale ou le choc hémorragique, le choc traumatique et autres, le cancer, par exemple les tumeurs solides ou le cancer lymphatique tel que les lymphomes des cellules T ou les leucémies des cellules T, les métastases ou l'angiogénèse, les maladies infectieuses, par exemple le choc toxique (par exemple induit par un superantigène), le choc septique, le syndrome de détresse respiratoire chez l'adulte ou les infections virales, par exemple le SIDA.

11. Utilisation selon la revendication 10, **caractérisée en ce que** ladite prévention ou ledit traitement comprend le rejet aigu ou chronique du greffon, ou les maladies inflammatoires ou auto-immunes.

12. Composé selon la revendication 1, de formule 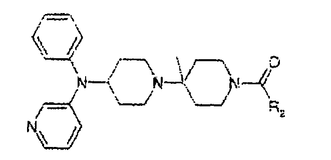 dans laquelle R₂ est de formule
